# EUROPEAN PATENT APPLICATION

(11) **EP 4 461 303 A2**
(43) Date of publication of application: **13.11.2024**
(21) Application number: 24184115.4
(22) Date of filing: 17.06.2020
(51) Int. Cl.: A61K 38/00

(54) **METHODS AND COMPOSITIONS FOR TREATING INFLAMMATORY CONDITIONS**

(30) Priority: 18.06.2019 US 201962862977 P
(62) Divisional of application: 20826563.7
(71) Applicant: The Scripps Research Institute, La Jolla, CA 92037 (US)
(72) Inventor: HEALY, Laura, San Diego, 92117 (US); MOSNIER, Laurent, San Diego, 92123 (US); GRIFFIN, John, Del Mar, 92014 (US)
(74) Representative: Hutter, Anton

(57) **Abstract**

The present invention provides novel compositions for suppressing inflammation and for treating inflammatory disorders. These compositions contain at least one PAR3-derived anti-inflammatory peptide or polypeptide and/or at least one PAR1-derived anti-inflammatory peptide or polypeptide. The PAR3- and/or PARI-derived peptides typically contain an amino acid sequence that mimics the respective N-terminal sequence of Activated Protein C-cleaved PAR3 or PARI, e.g., after activated protein C cleavage at residue Arg⁴¹ in human PAR3 and after residue Arg⁴⁶ in human PAR1. The invention also provides therapeutic methods of using the anti-inflammatory compositions described herein to suppress undesired inflammation and to treat inflammatory disorders. Additionally provided in the invention are methods of screening candidate compounds to identity novel anti-inflammatory agents.

## Description

### CROSS-REFERENCE TO RELATED APPLICATIONS

The subject patent application claims the benefit of priority to U.S. Provisional Patent Application Number 62/862,977 (filed June 18, 2019). The full disclosure of the priority application is incorporated herein by reference in its entirety and for all purposes.

### STATEMENT OF GOVERNMENT SUPPORT

This invention was made with government support under grant numbers HL052246, HL104165, and HL142975 awarded by the National Institutes of Health. The government has certain rights in the invention.

### BACKGROUND OF THE INVENTION

Inflammation is fundamentally a protective response through which the body responds to a variety of injuries, infections and stresses. Its ultimate goal is to eliminate the injury-inducing agent (that could be a microorganism, physical stimuli, chemical agent, etc.), prevent tissue damage and/or initiate the repair process. Inflammation can be local or systemic, and it can be acute or chronic. While the inflammatory response is critical for stress response, fending off infections and healing wounds, inflammation can also be damaging. Indeed, inflammation is an important component of the pathogenic process of many diseases and disorders. In addition, the presence of inflammation in many diseases, such as cancer, is indicative of a less favorable prognosis. Finally, in the extreme, inflammation may result in a life-threatening systemic response if not properly treated.

The serine protease activated protein C (APC), in addition to its traditional anticoagulant functions, exerts protective effects on a multitude of cell types and organs. APC can be anti-inflammatory via suppression of the NLRP3 inflammasome. The inflammasome plays a central role in innate immune system-driven inflammation, by promoting maturation and release of proinflammatory cytokines interleukin (IL)-1β and IL-18. Excess inflammation drives many diseases, including cardiovascular disease, suggesting that restricting inflammasome elaboration in the context of the innate immune system could be beneficial in human diseases. For example, in the Canakinumab Anti-inflammatory Thrombosis Outcome Study (CANTOS Study), an anti-IL-1β monoclonal antibody reduced overall nonfatal myocardial infarction, stroke and cardiovascular death.

The current methods for the treatment of inflammation are inadequate, and inflammation often remains untreated or is not treated effectively. As a consequence, considerable damage can be done to a subject with abnormal inflammation, and the subject's life can even be put in jeopardy. Thus, there is an unfulfilled need in the art for better or alternative means for countering inflammation and inflammatory disorders. The present invention addresses this and other unfulfilled needs in the art

### SUMMARY OF THE INVENTION

In one aspect, the present invention provides methods for suppressing an undesired inflammation and/or treating an inflammatory condition in a subject. These methods involve administering to the subject a pharmaceutical composition that contains an anti-inflammatory peptide derived from protease activated receptor-3 (PAR3). In various embodiments, the PAR3 derived anti-inflammatory peptide contains (1) an N-terminal fragment of Met¹-Arg⁴¹ deleted human PAR3 extracellular domain (SEQ ID NO:3), which consists of at least the first 4 N-terminal residues of SEQ ID NO:3, or conservatively modified variant thereof, or (2) at least 4 contiguous amino acid residues of human PAR3 derived peptide P3R (SEQ ID NO:4), which encompass Phe¹⁰-Phe¹² of SEQ ID NO:4, or conservatively modified variant thereof.

In some embodiments, the employed PAR3 derived anti-inflammatory peptide also possesses APC-like cytoprotective activities. In some embodiments, the employed PAR3 derived peptide contains at least the first 6, 7, 8, 9, 10, 11, 12, 13 or more N-terminal residues of SEQ ID NO:3. In some embodiments, the employed PAR3 derived peptide contains GAPPNSFEEFPFS (SEQ ID NO:8) or GAPPNSFEEFPFSALEGWTGATIT (SEQ ID NO:4). In some embodiments, the employed PAR3 derived peptide contains at least 6 contiguous amino acid residues of SEQ ID NO:4 encompassing Phe¹⁰-Phe¹². In some of these embodiments, the PAR3 derived peptide contains FPFSALEGW (SEQ ID NO: 10) or FPFSALEGWT GATIT (SEQ ID NO:9). In some embodiments, the employed PAR3 derived peptide is conjugated to a carrier moiety. For example, the carrier moiety can be a carrier protein, an immunoglobulin, a Fc domain, or a PEG molecule.

Some methods of the invention further involve administering to the subject an anti-inflammatory peptide derived from protease activated receptor-1 (PAR1). In various embodiments, the administered PAR1 derived peptide contains (1) at least the first 4 N-terminal residues of Met¹-Arg⁴¹ deleted human PAR1 extracellular domain (SEQ ID NO: 6) or a conservatively modified variant thereof, or (2) a variant of human PAR1 derived peptide TR47 (SEQ ID NO:7) with a deletion or substitution of at least one residue at its N-terminus. In some of these embodiments, the administered PAR1 derived peptide contains the first 4, 5, 6, 7, 8, 9, 10 or more N-terminal residues of human PAR1 derived peptide TR47 (SEQ ID NO:7). In some embodiments, the administered PAR1 derived peptide contains a TR47 variant having a substituted N-terminal residue. In some of these embodiments, the employed TR47 variant contains SEQ ID NO:49 (TR47ΔQ) or SEQ ID NO:50 (TR47ΔA). In some embodiments, the administered PAR1 derived peptide contains a TR47 variant having a deletion of one or more N-terminal residues. In some of these embodiments, the employed TR47 variant contains SEQ ID NO:47 (TR47(N-1)) or SEQ ID NO:48 (TR47(N-5)).

In some methods involving administration of both a PAR3 derived peptide and a PAR3 derived peptide, the two peptides are administered to the subject sequentially in any order. In some other methods, the two peptides are administered to the subject simultaneously. In some of these embodiments, a pharmaceutical composition containing both the PAR3 derived peptide and the PAR1 derived peptide is administered to the subject in need of treatment. In some of these embodiments, the PAR3 derived peptide is conjugated to the PAR1 derived peptide. For example, the PAR3 derived peptide can be covalently bonded to the PAR1 derived peptide (e.g., SEQ ID NO:61). In some of these embodiments, a linker moiety can be used for covalent linking the PAR3 derived peptide (e.g., SEQ ID NO:9) to the PAR1 derived peptide (e.g., SEQ ID NO:36) as exemplified herein. In some embodiments, the PAR3 derived peptide and/or the PAR1 derived peptide are conjugated to a carrier moiety. In some of these embodiments, the ratio of the PAR3-derived peptide and the PAR1-derived peptide is at least about 2:1, 4:1, 6:1, 8:1 or 10:1.

In some methods involving administration of both a PAR3 derived peptide and a PAR3 derived peptide, each of the PAR3 derived peptide and the PAR1 derived peptide is administered to the subject at a daily amount of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of the subject's body weight. In some other methods, the subject is administered with one peptide at a daily amount of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of the subject's body weight, and the other peptide at a daily amount of at least about 0.01 mg/kg, 0.025 mg/kg, 0.05 mg/kg, 0.1 mg/kg, 0.25 mg/ kg. 0.5 mg/kg, 1 mg/kg, 2.5 mg/kg, 5 mg/kg, 10 mg/kg, 25 mg/kg or higher, of the subject's body weight.

In various embodiments, the methods of the invention are directed to treating inflammatory disorders. The inflammatory disorders to be treated can be any one selected from the group consisting of asthma, autoimmune diseases, chronic inflammation, chronic prostatitis, gomerulonephritis, hypersensitivities, inflammatory bowel diseases, pelvic inflammatory diseases, reperfusion injury, rheumatoid arthritis, sterile inflammation, transplant rejection, virus-related inflammation, and vasculitis. In some embodiments, the methods of the invention are directed to treating a condition associated with undesired immune activation or undesired immune response. For example, the condition to be treated, which is associated with undesired immune activation or undesired immune response, can be a neuropathology, a viral infection, or malaria. In some methods, the subject to be treated with methods of the invention is afflicted with or suspected to have acute neuroinflammation, chronic neuroinflammation or malarial inflammation.

In another aspect, the invention provides a composition that contains a PAR3-derived anti-inflammatory peptide and a PAR1-derived anti-inflammatory peptide. In some of these embodiments, the PAR3 derived anti-inflammatory peptide contains (1) an N-terminal fragment of Met¹-Arg⁴¹ deleted human PAR3 extracellular domain (SEQ ID NO:3), which consists of at least the first 4 N-terminal residues of SEQ ID NO:3, or conservatively modified variant thereof, or (2) at least 4 contiguous amino acid residues of PAR3 derived peptide P3R (SEQ ID NO:4), which encompass Phe¹⁰-Phe¹² of SEQ ID NO:4, or conservatively modified variant thereof; and the PAR1 derived anti-inflammatory peptide contains (1) an N-terminal fragment of Met¹-Arg⁴⁶ deleted human PAR1 extracellular domain (SEQ ID NO:6) or conservatively modified variant thereof, wherein the fragment consists of at least the first 4 N-terminal residues of SEQ ID NO:6, or (2) a variant of human PAR1 derived peptide TR47 (SEQ ID NO:7) with a deletion or substitution of at least one residue at its N-terminus.

In some embodiments, the employed PAR3 derived peptide contains at least the first 13 N-terminal residues of SEQ ID NO:3 or conservatively modified variant thereof, and the PAR1 derived peptide comprises at least the first 8 N-terminal residues of SEQ ID NO:6 or conservatively modified variant thereof. In various embodiments, the employed PAR3 derived peptide contains SEQ ID NO:4, SEQ ID NO:8, SEQ ID NO:9, SEQ ID NO: 10 or conservatively modified variant thereof, and the PAR1 derived peptide contains SEQ ID NO:7, SEQ ID NO:35, SEQ ID NO:36, SEQ ID NO:43, SEQ ID NO:47, SEQ ID NO:49, SEQ ID NO:50 or conservatively modified variant thereof. In some compositions of the invention, the amount of the PAR3-derived peptide and the amount of the PAR1-derived peptide are at a ratio of at least about 2:1, 4:1, 6:1, 8:1 or 10: 1. In some compositions, the PAR3-derived peptide is conjugated to the PAR1-derived peptide. In some compositions, the PAR3-derived peptide is conjugated to the PAR1-derived peptide via a linker moiety or a carrier moiety. For example, a PAR1-derived peptide (e.g., SEQ ID NO:36) can be covalently bonded to a PAR3-derived peptide (e.g., SEQ ID NO:9) via a glycine linker, as exemplified in SEQ ID NO:61 herein. Some compositions of the invention are formulated for administration to the subject orally, intravenously, subcutaneously, intramuscularly, intranasally, intraocular, topical, or intraperitoneally.

Some compositions of the invention contain a daily dosage of each of the two peptides of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of average body weight of the subject group that the composition is intended for. Some other compositions of the invention contain (1) a daily dosage of one of the two peptides of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of average body weight of the subject group that the composition is intended for, and (2) a daily dosage of the other peptide of at least about 0.01 mg/kg, 0.025 mg/kg, 0.05 mg/kg, 0.1 mg/kg, 0.25 mg/ kg. 0.5 mg/kg, 1 mg/kg, 2.5 mg/kg, 5 mg/kg, 10 mg/kg, 25 mg/kg or higher, of average body weight of the subject group that the composition is intended for.

In another aspect, the invention provides methods for suppressing inflammation and treating inflammatory condition in a subject. These methods entail administering to the subject a pharmaceutical composition comprising a therapeutic effective amount of an anti-inflammatory peptide derived from protease activated receptor-1 (PAR1). In some of the methods, the administered PAR1 derived anti-inflammatory peptide contains (1) at least the first 4 N-terminal residues of Met¹-Arg⁴¹ deleted human PAR1 extracellular domain (SEQ ID NO:6) or a conservatively modified variant thereof, or (2) a variant of human PAR1 derived peptide TR47 (SEQ ID NO:7) with a deletion or substitution of at least one residue at its N-terminus. In some embodiments, the employed PAR1 derived peptide comprises the first 4, 5, 6, 7, 8, 9, 10 or more N-terminal residues of human PAR1 derived peptide TR47 (SEQ ID NO:7). In some methods, the employed PAR1 derived peptide contains a TR47 variant containing a substituted N-terminal residue. In some of these methods, the employed TR47 variant contains SEQ ID NO:49 (TR47ΔQ) or SEQ ID NO:50 (TR47ΔA). In some methods, the employed PAR1 derived peptide contains a TR47 variant containing a deletion of one or more N-terminal residues. In some these methods, the employed TR47 variant contains SEQ ID NO:47 (TR47(N-1)) or SEQ ID NO:48 (TR47(N-5)).

In still another aspect, the invention provides methods for identifying an anti-inflammatory agent. The methods require (1) culturing a group of human THP-1 cells, (2) contacting the cultured cells with an inflammation inducing agent, (3) contacting the cultured cells respectively with a candidate agent and a PAR3- or PAR1-derived anti-inflammatory peptide, (4) respectively measuring an inflammation related activity in cells that have been contacted with the candidate agent and cells that have been contacted with the PAR3- or PAR1-derived anti-inflammatory peptide. If a value of the inflammation activity measured in cells contacted with the candidate agent is the same or less than a value of the inflammation activity measured in cells contacted with the PAR3- or PAR1-derived anti-inflammatory peptide, the candidate agent is identified as an anti-inflammatory agent. In some methods, the employed PAR3- or PAR1-derived anti-inflammatory peptide is P3R (SEQ ID NO:4) or TR47 (SEQ ID NO:7), or a conservatively modified variant thereof. In various methods, the employed candidate agent is a peptide or polypeptide, variant, derivative or mimetic compound of a peptides or polypeptide that mimics the N-terminal sequence of Met¹-Arg⁴¹ deleted extracellular domain of human PAR3 (SEQ ID NO:3) or Met¹-Arg⁴⁶ deleted extracellular domain of human PAR1 (SEQ ID NO:6). In some methods, the inflammation related activity to be measured is caspase 1 enzymatic activity. In some other methods, the inflammation related activity to be measured is IL-1β release. In some methods, the employed inflammation inducing agent is lipopolysaccharide (LPS).

A further understanding of the nature and advantages of the present invention may be realized by reference to the remaining portions of the specification and claims.

### BRIEF DESCRIPTION OF THE DRAWINGS

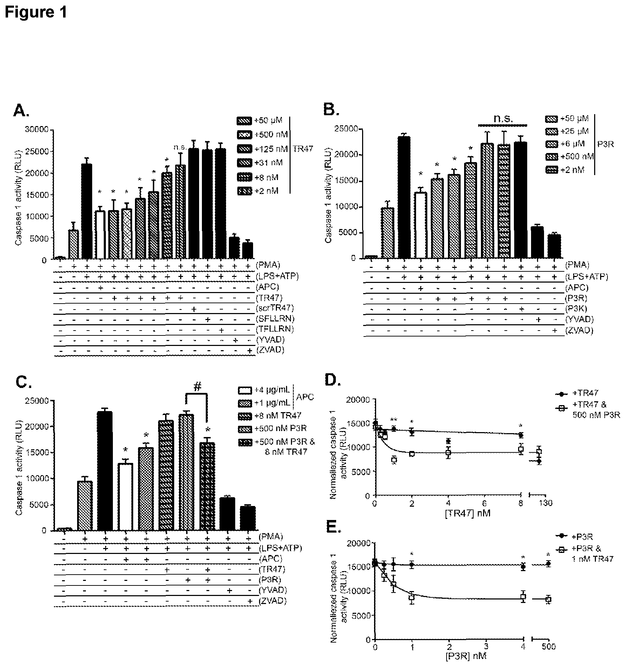

Figure 1 shows anti-inflammatory activity of PAR1-derived peptide TR47 and PAR3 derived peptide P3R. THP1-null (THP1) cells were plated at a final concentration of 1 x 10⁶/mL in 96 well plates and incubated with PMA (0.5 µM) for 3 hours 37°C in supplemented RPMI. Media was subsequently changed every 24 hours for 3 days. Various subsets of wells were selected and cells were treated for 60 minutes at 37°C in serum-free RPMI with: (A) APC (4 µg/mL), TR47 (50 µM - 2 nM), scrTR47 (50 µM), SFLLRN (50 µM), TFLLRN (50 µM); (B) P3R (50 µM - 500 nM), P3K (50 µM); (C) APC (4 or 1 µg/mL), TR47 (8 nM), P3R (500 nM) or TR47 + P3R (8 nM and 500 nM, respectively); (D) TR47 (0 - 128 nM) (●) ± P3R (500 nM) (□); (E) P3R (0 - 500 nM) (●) ± TR47 (1 nM) (□). Following DPBS wash, cells were incubated with LPS (1 µg/mL) for 3 hours at 37°C in serum-free RPMI. After a DPBS wash, cells were incubated with ATP (5 mM) in the presence or absence of 10 µM YVAD (caspase 1 inhibitor) or 2.5 µM ZVAD (pan-caspase inhibitor) for 45 minutes at 37°C, prior to performing the caspase 1 activity assay following manufacturer's directions measuring luminescence (relative luminescent units, RLU). (D & E) Experimental caspase 1 activity was normalized to YVAD (caspase 1 inhibitor) luminescence units. (A-E) Data points represent mean ± S.D. of at least 3 independent experiments. *P < 0.05 (vs. LPS + ATP); #P < 0.05 (vs. LPS+ATP & APC, TR47 or P3R); **P <0.005 (vs. LPS+ATP & TR47 + 500 nM P3R); n.s. not significant.

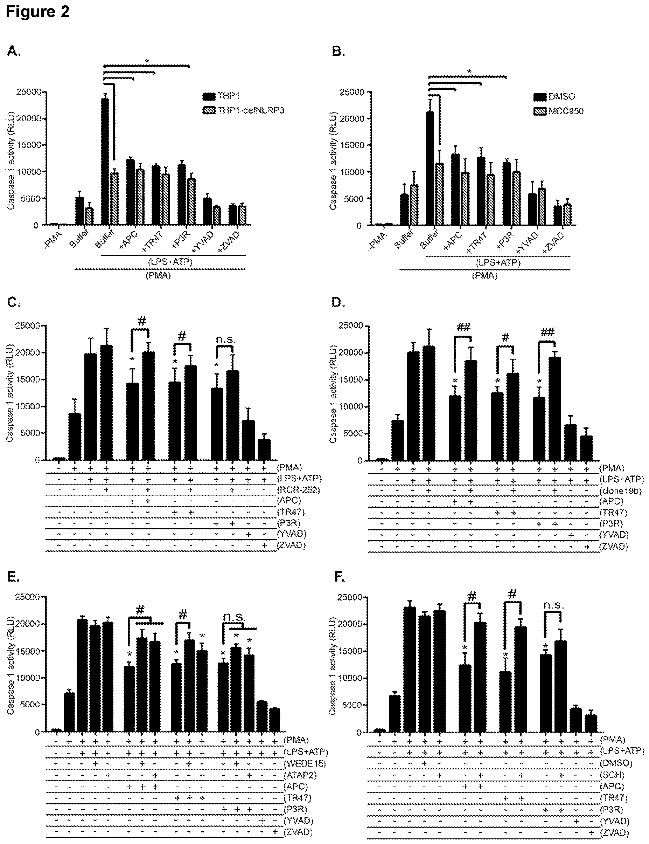

Figure 2 shows that APC and PAR1-derived and PAR3-derived peptides reduce NLRP3-driven inflammasome caspase 1 activity in part via EPCR, PAR1 and PAR3. THP1 or (A) NLRP3 deficient cells (THP1-defNLRP3) cells were plated at a final concentration of 1 × 10⁶/mL in 96 well plates and incubated with PMA (0.5 µM) for 3 hours 37°C in supplemented RPMI. Media was subsequently changed every 24 hours for 3 days. In brief, selected wells were treated with APC (4 µg/mL), TR47 (50 µM) or P3R (50 µM) for 1 hour, washed with DPBS, and incubated with LPS (1 µg/mL) for 3 hours at 37°C in serum-free RPMI. After a DPBS wash, cells were incubated with ATP (5 mM) in the presence or absence of 10 µM YVAD (caspase 1 inhibitor) or 2.5 µM ZVAD (pan-caspase inhibitor) for 45 minutes at 37°C. In (B), after PMA differentiation, select wells were treated with APC (4 µg/mL), TR47 (50 µM) or P3R (50 µM) for 1 hour, washed with DPBS, and incubated with LPS (1 µg/mL) for 3 hours at 37°C in serum-free RPMI. Cells were washed with DPBS before being treated with the NLRP3 inhibitor MCC950 (10 µM) or DMSO for 30 minutes. Again, cells were washed with DPBS and incubated with ATP (5 mM) in the presence or absence of 10 µM YVAD (caspase 1 inhibitor) or 2.5 µM ZVAD (pan-caspase inhibitor) for 45 minutes prior to performing the caspase 1 activity following manufacturer's directions measuring luminescence. In (C-F) prior to APC, TR47 or P3R treatment, cells were incubated with antibodies or inhibitors. In (C) cell were incubated with the blocking EPCR antibody, RCR-252 (50 µg/mL); (D) mouse monoclonal antibody to PAR3, clone 19b (clone19b; 25 µg/mL) for 30 minutes. In (E) cells were treated with either mouse monoclonal antibodies to PAR1, WEDE15 (20 µg/mL) or ATAP2 (10 µg/mL) for 15 minutes. In (F) cells were treated with the small molecule PAR1 inhibitor, SCH79797 (SCH; 20 µM) for 20 minutes. Following antibody or small molecule treatments (C-F) selected wells were treated with APC (4 µg/mL), TR47 (50 µM) or P3R (50 µM) for 1 hour, washed with DPBS, and incubated with LPS (1 µg/mL) for 3 hours at 37°C in serum-free RPMI. After a DPBS wash, cells were incubated with ATP (5 mM) in the presence or absence of 10 µM YVAD (caspase 1 inhibitor) or 2.5 µM ZVAD (pan-caspase inhibitor) for 45 minutes at 37°C, prior to performing the caspase 1 activity assay following manufacturer's directions measuring luminescence (relative luminescent units, RLU). (A-F) Data points represent mean ± SD of at least 3 independent experiments. *P < 0.05 (vs. LPS + ATP); #P < 0.05 (vs. LPS+ATP & APC, TR47 or P3R); ##P <0.005 (vs. LPS+ATP & APC, TR47 or P3R); n.s. not significant.

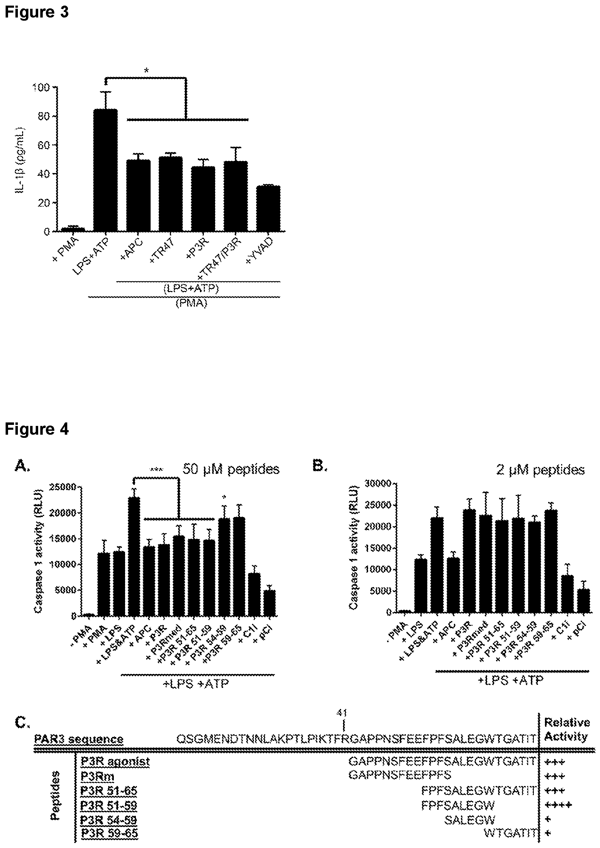

Figure 3 shows that APC and PAR1-derived and PAR3-derived peptides reduce IL-1β release. THP1 cells were plated at a final concentration of 1 × 10⁶/mL in 96 well plates and incubated with PMA (0.5 µM) for 3 hours 37°C in supplemented RPMI. Media was subsequently changed every 24 hours for 3 days. Selected wells were treated with APC (4 µg/mL), TR47 (50 µM), P3R (50 µM), or TR47/P3R (8 nM & 500 nM, respectively) for 1 hour, washed with DPBS, and incubated with LPS (1 µg/mL) for 3 hours at 37°C in serum-free RPMI. Cells were washed with DPBS and incubated with ATP (5 mM) in the presence or absence of 10 µM YVAD (caspase 1 inhibitor) for 45 minutes, and the resulting cell supernatant was used in the IL-1β Quantikine ELISA following manufacturer's (R&D) directions measuring absorbance, and the concentration of IL-1β was calculated following measurement of the standard curve. Data points represent mean ± SEM of at least 3 independent experiments. *P < 0.05 (vs. LPS + ATP).
Figure 4 shows anti-inflammatory activities of various fragments of PAR3-derived peptide P3R. THP1 cells were plated at a final concentration of 1 × 10⁶/mL in 96 well plates and incubated with PMA (0.5 µM) for 3 hours 37°C in supplemented RPMI. Media was subsequently changed every 24 hours for 3 days. Selected wells were treated with APC (4 µg/mL), (A) 50 µM or (B) 2 µM of P3R, P3Rm, P3R 51-65, P3R 51-59, P3R 54-59 or P3R 59-65 for 1 hour, washed with DPBS, and incubated with LPS (1 µg/mL) for 3 hours at 37°C in serum-free RPMI. Cells were washed with DPBS and incubated with ATP (5 mM) in the presence or absence of 10 µM YVAD (caspase 1 inhibitor; C1i) or 2.5 µM ZVAD (pan-caspase inhibitor; pCi) for 45 minutes at 37°C, prior to performing the caspase 1 activity assay following manufacturer's directions measuring luminescence (relative luminescent units, RLU). (C) Diagram showing the PAR3 sequence, peptide sequences and relative (anti-inflammatory) activity. These include sequences of P3R (SEQ ID NO:4), P3Rm (SEQ ID NO:8), P3R 51-65 (SEQ ID NO:9), P3R 51-59 (SEQ ID NO:10), P3R 54-59 (SEQ ID NO:54) and P3R 59-65 (SEQ ID NO:55).

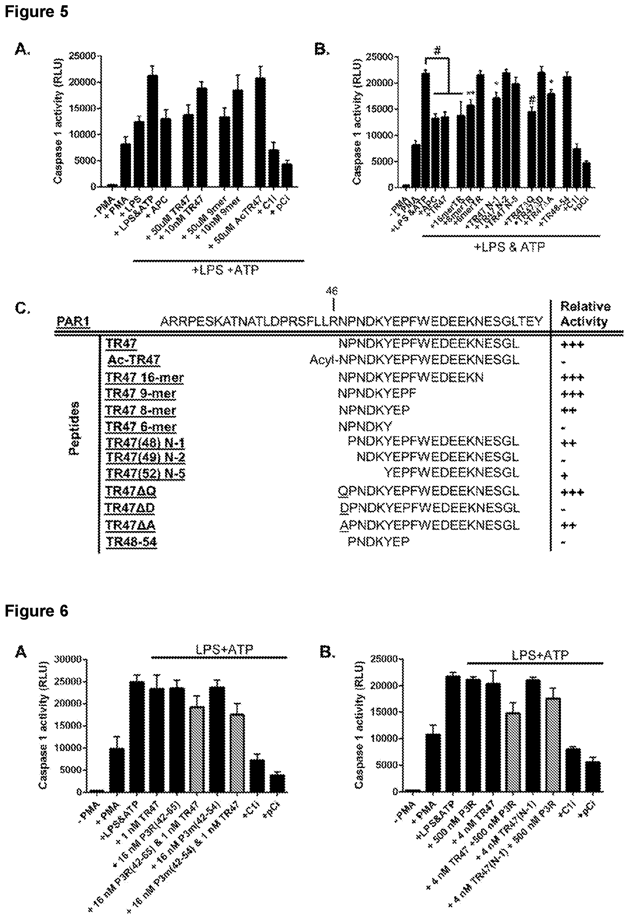

Figure 5 shows anti-inflammatory activities of variants of PAR1-derived peptide TR47. THP1 cells were plated at a final concentration of 1 × 10⁶/mL in 96 well plates and incubated with PMA (0.5 µM) for 3 hours 37°C in supplemented RPMI. Media was subsequently changed every 24 hours for 3 days. Selected wells were treated with APC (4 µg/mL), TR47, in (A) 9-mer (TR47 9-mer; NPNDKYEPF (SEQ ID NO:36)) or Ac-TR47 (Acylated TR47) at either 50 µM or 10 nM or (B) 50 µM of 16-mer-TR47, 8-mer-TR47, 6-mer-TR47, TR47 N-1, TR47 N-2, TR47 N-5, TR47ΔQ, TR47ΔD, TR47ΔA or TR48-54 for 1 hour, washed with DPBS, and incubated with LPS (1 µg/mL) for 3 hours at 37°C in serum-free RPMI. Cells were washed with DPBS and incubated with ATP (5 mM) in the presence or absence of 10 µM YVAD (caspase 1 inhibitor; C1i) or 2.5 µM ZVAD (pan-caspase inhibitor; pCi) for 45 minutes at 37°C, prior to performing the caspase 1 activity assay following manufacturer's directions measuring luminescence (relative luminescent units, RLU). # P < 0.001 (vs. LPS+ATP), ** P <0.005 (vs. LPS+ATP), * (P <0.05 vs. LPS+ATP). (C) Diagram showing the PAR1 sequence, peptide sequences and relative (anti-inflammatory) activity. These include sequences of TR47 (SEQ ID NO:7), Ac-TR47 (SEQ ID NO:56), 16-mer-TR47 (SEQ ID NO:43), 9-mer-TR47 (SEQ ID NO:36), 8-mer-TR47 (SEQ ID NO:35), 6-mer-TR47 (SEQ ID NO:33), TR47 N-1 (SEQ ID NO:47), TR47 N-2 (SEQ ID NO:51), TR47 N-5 (SEQ ID NO:48), TR47ΔQ (SEQ ID NO:49), TR47ΔD (SEQ ID NO:52), TR47ΔA (SEQ ID NO:50) and TR48-54 (SEQ ID NO:53).
Figure 6 shows that PAR3-derived and PARI-derived peptide variants exhibit cooperativity to reduce caspase 1 activity. THP1 cells were plated at a final concentration of 1 × 10⁶/mL in 96 well plates and incubated with PMA (0.5 µM) for 3 hours 37°C in supplemented RPMI. Media was subsequently changed every 24 hours for 3 days. In (A) select wells were treated with P3(42-65) or P3(42-54) (0 or 16 nM) in the presence or absence of TR47 (1 nM) for 60 min at 37°C. In (B) select wells were treated with TR47 (0 or 4 nM) or TR47(N-1) (0 or 4 nM) in the presence or absence of 500 nM P3R for 60 min at 37°C. Cells were washed with DPBS, and incubated with LPS (1 µg/mL) for 3 hours at 37°C in serum-free RPMI. Cells were washed with DPBS and incubated with ATP (5 mM) in the presence or absence of 10 µM YVAD (caspase 1 inhibitor; C1i) or 2.5 µM ZVAD (pan-caspase inhibitor; pCi) for 45 minutes at 37°C, prior to performing the caspase 1 activity assay following manufacturer's directions measuring luminescence (relative luminescent units, RLU).

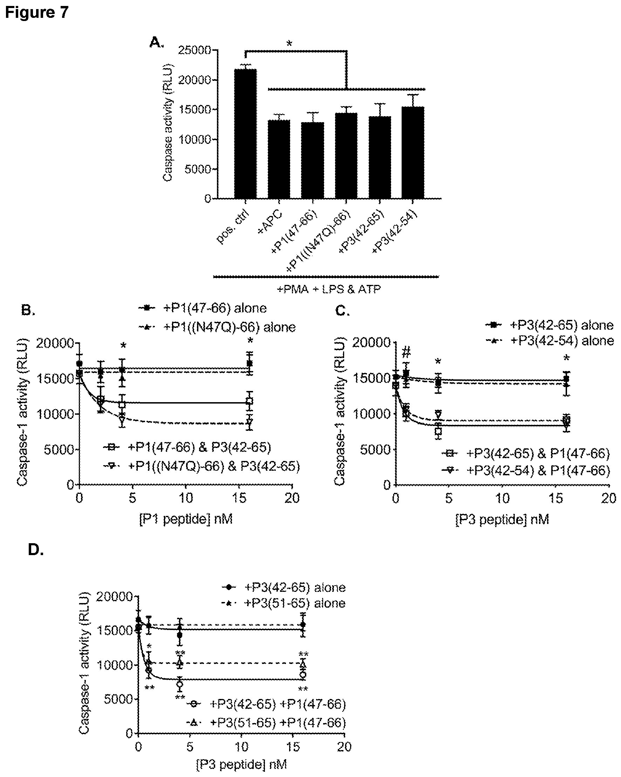

Figure 7 shows that variants of either a PAR1-derived peptide or a PAR3-derived peptide cooperatively reduce caspase-1 activity. THP-1-null (THP-1 from Invivogen) cells were plated at a final concentration of 1 × 10⁶/mL in 96 well plates and incubated with PMA (0.5 µM) for 3 hours 37°C in supplemented RPMI. Media was subsequently changed every 24 hours for 3 days. Subsets of wells were selected and cells were treated for 60 minutes at 37°C in serum-free RPMI with: (A) APC (4 µg/mL) or with 50 µM of peptides including "TR47" (PAR1(47-66); SEQ ID NO:7), "TR47ΔQ" (PAR1(N47Q)-66; SEQ ID NO:49), "P3R" (PAR3(42-65); SEQ ID NO:4), or "P3Rm" (PAR3(42-54); SEQ ID NO:8) as indicated); (B) TR47 (0 - 16 nM) (■) alone or with P3R (500 nM) (□) or TR47ΔQ (0-16 nM) (▲) alone or with P3R (500 nM) (∇); (C) P3R (0 - 16 nM) (■) alone or with TR47 (1 nM) (□) or P3Rm (0-16 nM) (▲) alone or with TR47 (1 nM) (∇); or (D) Subsets of wells were selected and cells were treated for 60 minutes at 37°C in serum-free RPMI with P3R (0 - 16 nM) (●) ± TR47 (1 nM) (○) or P3R51-65 (SEQ ID NO:9) (0-16 nM) (▲; dotted line) ± TR47 (1 nM) (△; dotted line). Following DPBS wash, cells were incubated with LPS (1 µg/mL) for 3 hours at 37°C in serum-free RPMI. After a DPBS wash, cells were incubated with ATP (5 mM) in the presence or absence of 10 µM YVAD (caspase-1 inhibitor) or 2.5 µM ZVAD (pan-caspase inhibitor) for 45 minutes at 37°C, prior to performing the caspase-1 activity assay following manufacturer's directions measuring luminescence (relative luminescent units, RLU). Experimental caspase-1 activity was normalized to the value for YVAD (caspase-1 inhibitor) luminescence units. (B), (C), and (D): Experimental caspase-1 activity was normalized to the value for YVAD (caspase-1 inhibitor) luminescence units. For (A-C): Data points represent mean ± S.D. of at least 3 independent experiments. *P < 0.005 (vs. LPS + ATP or vs. LPS+ATP and TR47 or P3R alone or variant); #P < 0.05 (vs. LPS+ATP and P3R alone or P3(42-54) alone); n.s. not significant. For (D), *P < 0.05 (vs. LPS+ATP and P3 peptide alone); **P <0.005 (LPS+ATP & P3 peptide alone); n.s. not significant.

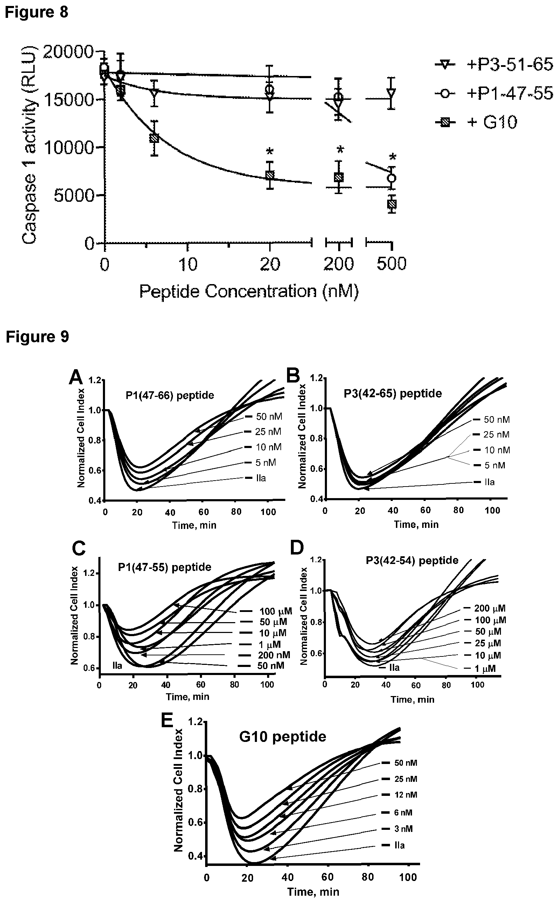

Figure 8 shows that a PAR1 :PAR3 covalently linked peptide reduces caspase-1 activity. THP-1 cells were plated at a final concentration of 1 × 10⁶/mL in 96 well plates and incubated with PMA (0.5 µM) for 3 hours 37°C in supplemented RPMI. Media was subsequently changed every 24 hours for 3 days. Selected wells were treated with PAR1 9-mer peptide P1(47-55) (SEQ ID NO:36) (0 - 500 nM) (O), PAR3 peptide P3(51-65) (SEQ ID NO:9) (0 - 500 nM) (∇) or a Gly10-linked PAR1/PAR3 fusion peptide "G10" (0 - 500 nM) (□) for 60 minutes at 37°C. Cells were washed with DPBS, and incubated with LPS (1 µg/mL) for 3 hours at 37°C in serum-free RPMI. Cells were washed with DPBS and incubated with ATP (5 mM) in the presence or absence of 10 µM YVAD (caspase-1 inhibitor) for 45 minutes at 37°C, prior to performing the caspase-1 activity assay following manufacturer's directions measuring luminescence (relative luminescent units, RLU), whereupon experimental caspase-1 activity was adjusted to Caspase-1 inhibitor luminescence units and expressed as Caspase-1 activity. Data points represent mean ± S.D. of at least 3 independent experiments. *P < 0.05 (vs. LPS + ATP).
Figure 9 shows that PAR1-derived, PAR3-derived and PAR1/PAR3 fusion peptides inhibit thrombin-induced disruption of the endothelial barrier. The bottom curve in each panel of the figure is labeled as IIa for thrombin alone and represents the observed effects for thrombin (IIa) in the absence of peptide. In each panel, IIa was always present for each peptide added. The number in each panel indicates the concentration of each peptide for the respective curves. The peptide concentration of 0 is represented by the bottom curve labeled "IIa". (A) the dose response inhibition over time of TER, expressed by Normalized Cell Index (NCI) by the PAR1 derived peptide P1(47-66) or TR47 using concentrations between 5 and 50 nM. (B) the dose response inhibition over time of NCI by the PAR3-derived peptide P3(42-65) or P3R using a 5 to 50 nM concentration range. (C) The NCI dose response inhibition over time by the short sequence PAR1-derived peptide P1(47-55) using a range of concentrations between 50 nM and 100 µM peptide. (D) The NCI dose response inhibition over time by the short sequence PAR3-derived peptide P3(42-54) using a range of concentrations between 1 µM and 200 µM peptide. (E) The NCI dose response inhibition over time by the hybrid peptide G10 in which the P1(47-55) peptide described in panel C was linked to a PAR3 peptide P3(51-65) by using 10 Gly residues. In this case the range of concentrations to study the dose response inhibition was between 3 and 50 nM peptide. An estimate for the percentage of activity for each peptide dilution in presence of thrombin can be quantified by obtaining the Area under the Curve (AUC) for each peptide dilution in comparison to the value for the AUC for thrombin alone in the absence of any peptide. These values were used to calculate the potency of each PAR peptide.

### DETAILED DESCRIPTION OF THE INVENTION

### I. Overview

The present invention relates to anti-inflammatory activities of various peptides or polypeptides that are derived from PAR1 and PAR3. The invention is presided in part on the discovery by the inventors that some peptides mimicking the N-terminal sequences of PAR3 and PAR1 that are cleavage by APC at the respective noncanonical sites possess anti-inflammatory activities. Surprisingly, it was also discovered there is a strong synergistic effect in the observed anti-inflammatory activities between the PAR3-derived and the PAR1-derived peptides.

Activated Protein C (APC) is a protease that cleaves PAR1 at both Arg⁴¹ and Arg⁴⁶. APC cleaves PAR3 at Arg⁴¹. APC cleavage is known to be cytoprotective and anti-apoptotic. At the heart of this invention is the following discovery. Based on cleavage by APC of the peptide bond between Arg⁴⁶ and Asn⁴⁷ in PARl, peptides mimicking partial sequences of PARl that begin with the N-terminal residue Asn⁴⁷, such as residues 47-NPNDKYEPFWEDEEKNESGL-66 ("TR47" or "P1(47-66)), cause anti-inflammatory cell signaling which often can involve suppression of the inflammasome. In addition, based on APC cleavage of the peptide bond between Arg⁴¹ and Gly⁴² in PAR3, peptides mimicking partial sequences of PAR3 that begin with the N-terminal residue Gly⁴² such as residues 42-GAPPNSFEEFPFSALEGWTGATIT-65 ("P3R"), also cause anti-inflammatory cell signaling.

As detailed herein, the inventors delineated a role for EPCR, PAR1 and PAR3 in potentiating the anti-inflammatory activity of APC using an induced human macrophage-like cell line. The inventors discovered that TR47, a PAR1-derived peptide, and P3R, a PAR3-derived peptide, each is anti-inflammatory alone and that combinations of these two peptides can synergistically exert anti-inflammatory effects. Specifically, the inventors assessed the relevance of APC suppression of the inflammasome in the human immune system by using the THP1 model cell line, used historically for studying both the inflammasome and APC's cytoprotective activity. Caspase-1, a cysteine protease in the NLRP3 inflammasome, is diagnostic for inflammasome activation. The inventors monitored caspase-1 activity of activated THP1 cells to evaluate APC's anti-inflammatory actions on human cells. The inventors further evaluated whether protease activated receptor (PAR1- and PAR3-derived peptides that mimic APC's non-canonical cleavages in these two G-protein-coupled-receptors (GPCRs), namely TR47 and P3R, respectively, exert anti-inflammatory activity in human THP1 cells. It was found that in the human THP1 cell line, APC, TR47, and P3R each can restrict the NLRP3 inflammasome as measured by caspase-1 activity and IL-1β release. Surprisingly, it was observed that the two peptides can synergistically suppress inflammasome activity. The anti-inflammatory effects of the PAR peptides point towards their utilities as novel anti-inflammatory pharmacological agents.

To further demonstrate the observed synergistic effect, the inventors also generated a PAR1/PAR3 fusion peptide by covalently linking a PAR1-derived peptide and a PAR3-derived peptide, and examined its anti-inflammatory efficacy. Not surprisingly, it was found that the covalently linked PAR1/PAR3 peptide significantly reduced caspase-1 activity at a concentration whereas each of the component peptides alone had less of an anti-inflammatory effect. Importantly, it was also observed that the covalently linked PAR1/PAR3 fusion peptide also inhibits thrombin-induced decrease of transendothelial electrical resistance (TEER). These data provide further evidence that the PAR1- and PAR3-derived peptides can exert anti-inflammatory activities and cytoprotective activities (e.g., preventing thrombin-induced endothelial barrier disruption) in a synergistic manner.

In accordance with these discoveries, the present invention provides novel methods of inhibiting or suppressing inflammation, and methods for treating inflammatory disorders or conditions. Therapeutic compositions that can be used for carrying out the therapeutic methods of the invention are also provided herein.

Unless otherwise stated, the present invention can be performed using standard procedures, as described, for example in Methods in Enzymology, Volume 289: Solid-Phase Peptide Synthesis, J. N. Abelson, M. I. Simon, G. B. Fields (Editors), Academic Press; 1st edition (1997) (ISBN-13: 978-0121821906); U.S. Pat. Nos. 4,965,343, and 5,849,954; Maniatis et al., Molecular Cloning: A Laboratory Manual, Cold Spring Harbor Laboratory Press, Cold Spring Harbor, N.Y., USA (1982); Sambrook et al., Molecular Cloning: A Laboratory Manual (2nd ed.), Cold Spring Harbor Laboratory Press, Cold Spring Harbor, N.Y., USA (1989); Davis et al., Basic Methods in Molecular Biology, Elsevier Science Publishing, Inc., New York, USA (1986); or Methods in Enzymology: Guide to Molecular Cloning Techniques Vol. 152, S. L. Berger and A. R. Kimmerl Eds., Academic Press Inc., San Diego, USA (1987); Current Protocols in Protein Science (CPPS) (John E. Coligan, et. al., ed., John Wiley and Sons, Inc.), Current Protocols in Cell Biology (CPCB) (Juan S. Bonifacino et. al. ed., John Wiley and Sons, Inc.), and Culture of Animal Cells: A Manual of Basic Technique by R. Ian Freshney, Publisher: Wiley-Liss; 5th edition (2005), Animal Cell Culture Methods (Methods in Cell Biology, Vol. 57, Jennie P. Mather and David Barnes editors, Academic Press, 1st edition, 1998). The following sections provide additional guidance for practicing the compositions and methods of the present invention.

### II. Definitions

Unless defined otherwise, all technical and scientific terms used herein have the same meaning as commonly understood by those of ordinary skill in the art to which this invention pertains. The following references provide one of skill with a general definition of many of the terms used in this invention: Oxford Dictionary of Biochemistry and Molecular Biology, Smith et al. (eds.), Oxford University Press (revised ed., 2000); Dictionary of Microbiology and Molecular Biology, Singleton et al. (Eds.), John Wiley & Sons (3PrdP ed., 2002); and A Dictionary of Biology (Oxford Paperback Reference), Martin and Hine (Eds.), Oxford University Press (4PthP ed., 2000). In addition, the following definitions are provided to assist the reader in the practice of the invention.

The singular terms "a," "an," and "the" include plural referents unless the context clearly indicates otherwise. Similarly, the word "or" is intended to include "and" unless the context clearly indicates otherwise.

As used herein, the term "amino acid" of a peptide refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an a carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. The PAR1 derived protective polypeptides of the invention encompass derivative or analogs which have be modified with non-naturally coding amino acids.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of" refers to those elements required for a given embodiment. The term permits the presence of elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

The term "conservatively modified variant" or "conservatively substituted variant" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" refer to a variant which has conservative amino acid substitutions, amino acid residues replaced with other amino acid residue having a side chain with a similar charge. Families of amino acid residues having side chains with similar charges have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

As used herein, a "derivative" of a reference molecule (e.g., an anti-inflammatory peptide disclosed herein) is a molecule that is chemically modified relative to the reference molecule while substantially retaining the biological activity. The modification can be, e.g., oligomerization or polymerization, modifications of amino acid residues or peptide backbone, cross-linking, cyclization, conjugation, fusion to additional heterologous amino acid sequences, or other modifications that substantially alter the stability, solubility, or other properties of the peptide.

The terms "decrease" , "reduced", "reduction" , "decrease" or "inhibit" are all used herein generally to mean a decrease by a statistically significant amount. However, for avoidance of doubt, ""reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (e.g. absent level as compared to a reference sample), or any decrease between 10-100% as compared to a reference level.

The term "engineered cell" or "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The term "fragment" refers to any peptide or polypeptide having an amino acid residue sequence shorter than that of a full-length polypeptide whose amino acid residue sequence is described herein. An isolated peptide of PAR1 is shortened or truncated compared to its parent full-length PAR1. Relative to a full length PAR1 sequence, the PAR3 or PAR1 derived polypeptides or peptides of the invention typically have N-terminus truncation after the conserved Arg⁴¹ and Arg⁴⁶ residue, respectively, and possibly with additional N-terminal deletions or substitutions as described herein. These fragments can additionally contain C-terminus truncations (e.g., truncations of up to 50, 100, 200, 300 or more C-terminal residues) and/or also internal deletions.

"Inflammation" or "inflammatory response" refers to an innate immune response that occurs when tissues are injured by bacteria, trauma, toxins, heat, or any other cause. The damaged tissue releases compounds including histamine, bradykinin, and serotonin. Inflammation refers to both acute responses (i.e., responses in which the inflammatory processes are active) and chronic responses (i.e., responses marked by slow progression and formation of new connective tissue). Acute and chronic inflammation can be distinguished by the cell types involved. Acute inflammation often involves polymorphonuclear neutrophils; whereas chronic inflammation is normally characterized by a lymphohistiocytic and/or granulomatous response. Inflammation includes reactions of both the specific and non-specific defense systems. A specific defense system reaction is a specific immune system reaction response to an antigen (possibly including an autoantigen). A non-specific defense system reaction is an inflammatory response mediated by leukocytes incapable of immunological memory. Such cells include granulocytes, macrophages, neutrophils and eosinophils.

Inflammatory disorders are diseases caused by abnormally regulated inflammatory response, e.g., rheumatoid arthritis, hay fever, and atherosclerosis. Inflammation or inflammatory response refers to an innate immune response that occurs when tissues are injured by bacteria, trauma, toxins, heat, or any other cause. The damaged tissue releases compounds including histamine, bradykinin, and serotonin. Inflammation includes both acute responses (i.e., responses in which the inflammatory processes are active) and chronic responses (i.e., responses marked by slow progression and formation of new connective tissue). Acute and chronic inflammation can be distinguished by the cell types involved. Acute inflammation often involves polymorphonuclear neutrophils; whereas chronic inflammation is normally characterized by a lymphohistiocytic and/or granulomatous response. Inflammation includes reactions of both the specific and non-specific defense systems. A specific defense system reaction is a specific immune system reaction response to an antigen (possibly including an autoantigen). A non-specific defense system reaction is an inflammatory response mediated by leukocytes incapable of immunological memory. Such cells include granulocytes, macrophages, neutrophils and eosinophils.

The term "isolated" means the protein is removed from its natural surroundings. However, some of the components found with it may continue to be with an "isolated" protein. Thus, an "isolated polypeptide" is not as it appears in nature but may be substantially less than 100% pure protein.

The terms "identical" or percent "identity," in the context of two or more nucleic acids or polypeptide sequences, refer to two or more sequences or subsequences that are the same. Two sequences are "substantially identical" if two sequences have a specified percentage of amino acid residues or nucleotides that are the same (*i.e.,* 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

Methods of alignment of sequences for comparison are well known in the art. Optimal alignment of sequences for comparison can be conducted, e.g., by the local homology algorithm of Smith and Waterman, Adv. Appl. Math. 2:482c, 1970; by the homology alignment algorithm of Needleman and Wunsch, J. Mol. Biol. 48:443, 1970; by the search for similarity method of Pearson and Lipman, Proc. Nat'l. Acad. Sci. USA 85:2444, 1988; by computerized implementations of these algorithms (GAP, BESTFIT, FASTA, and TFASTA in the Wisconsin Genetics Software Package, Genetics Computer Group, Madison, WI); or by manual alignment and visual inspection (see, e.g., Brent et al., Current Protocols in Molecular Biology, John Wiley & Sons, Inc. (ringbou ed., 2003)). Two examples of algorithms that are suitable for determining percent sequence identity and sequence similarity are the BLAST and BLAST 2.0 algorithms, which are described in Altschul et al., Nuc. Acids Res. 25:3389-3402, 1977; and Altschul et al., J. Mol. Biol. 215:403-410, 1990, respectively.

Other than percentage of sequence identity noted above, another indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

Unless otherwise specified, the terms "polypeptide" and "peptide" are used interchangeably herein (e.g., "PAR3 derived anti-inflammatory polypeptide" and "PAR1 derived anti-inflammatory peptide") to refer to a polymer of amino acid residues. They encompass both short oligopeptides (e.g., peptides with less than about 25 residues) and longer polypeptide molecules (e.g., polymers of more than about 25 or 30 amino acid residues). Typically, the anti-inflammatory peptides (oligopeptides) or polypeptides of the invention can comprise from about 4 amino acid residues to about 350 or more amino acid residues in length. In some embodiments, the peptides or polypeptides comprise from about 8 amino acid residues to about 60 amino acid residues in length. The anti-inflammatory peptides or polypeptides of the invention include naturally occurring amino acid polymers and non-naturally occurring amino acid polymer, as well as amino acid polymers in which one or more amino acid residue is an artificial chemical mimetic of a corresponding naturally occurring amino acid. Unless otherwise indicated, a particular polypeptide sequence also implicitly encompasses conservatively modified variants thereof.

As used herein, the term "peptide mimetic" or "peptidomimetic" refers to a derivative compound of a reference peptide (e.g., an anti-inflammatory polypeptide disclosed herein) that biologically mimics the peptide's functions. Typically, the peptidomimetic derivative of a PAR1 derived anti-inflammatory polypeptide of the invention has at least 50%, at least 75% or at least 90% of the biological activities (e.g., inhibition of caspase 1 activity) of the reference polypeptide.

The term "operably linked" refers to a functional relationship between two or more polynucleotide (e.g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis-acting. However, some transcriptional regulatory sequences, such as enhancers, need not be physically contiguous or located in close proximity to the coding sequences whose transcription they enhance.

As used herein, the term "orthologs" or "homologs" refers to polypeptides that share substantial sequence identity and have the same or similar function from different species or organisms. For example, PAR3 and PAR1, respectively, from human, rabbit, rat, mouse and many other animal species are orthologs due to the similarities in their sequences and functions.

The phrase "signal transduction pathway" or "signaling activities" (e.g., APC, PAR3 or PAR1 mediated cytoprotective signaling) refers to at least one biochemical reaction, but more commonly a series of biochemical reactions, which result from interaction of a cell with a stimulatory compound or agent. Thus, the interaction of a stimulatory compound (e.g., a PAR3- or PAR1- derived peptide) with a cell generates a "signal" that is transmitted through the signal transduction pathway, ultimately resulting in a cellular response.

The term "subject" includes human and non-human animals. Non-human animals include all vertebrates, e.g., mammals and non-mammals, such as non-human primates, sheep, dog, cow, chickens, amphibians, and reptiles. Except when noted, the terms "patient" or "subject" are used herein interchangeably.

The term "agent" includes any substance, molecule, element, compound, entity, or a combination thereof. It includes, but is not limited to, e.g., protein, polypeptide, small organic molecule, polysaccharide, polynucleotide, and the like. It can be a natural product, a synthetic compound, or a chemical compound, or a combination of two or more substances. Unless otherwise specified, the terms "agent", "substance", and "compound" are used interchangeably herein.

Administration "in conjunction with" one or more other therapeutic agents includes simultaneous (concurrent) and consecutive administration in any order.

The term "contacting" has its normal meaning and refers to combining two or more agents (e.g., polypeptides or small molecule compounds) or combining agents and cells. Contacting can occur in vitro, e.g., combining two or more agents or combining an agent and a cell or a cell lysate in a test tube or other container. Contacting can also occur in a cell or in situ, e.g., contacting two polypeptides in a cell by coexpression in the cell of recombinant polynucleotides encoding the two polypeptides, or in a cell lysate. Contacting can also occur inside the body of a subject, e.g., by administering to the subject an agent which then interacts with the intended target (e.g., a tissue or a cell).

Protease-activated receptors (PARs) are a subfamily of related G protein-coupled receptors that are activated by cleavage of part of their extracellular domain. They are highly expressed in platelets, and also on endothelial cells, myocytes and neurons. There are 4 known protease-activated receptors, PAR1, PAR2, PAR3, and PAR4. They are members of the seven-transmembrane G-protein-coupled receptor superfamily. PARs are activated by the action of serine proteases, such as thrombin (acts on PARs 1, 3 and 4), activated protein C (on PARs 1, 2, and 3), trypsin (on PAR 2), or other proteases, at the N terminus to reveal a tethered ligand. The rate-limiting step of PAR signaling is determined by the efficiency of proteolysis of the N terminus, which is regulated by allosteric binding sites, cofactors, membrane localization, and receptor dimerization. This ultimately controls the initiation of PAR signaling. In addition, these factors also control the cellular response by directing signaling toward G-protein or β-arrestin pathways. PAR1 signaling on endothelial cells is controlled by the activating protease and heterodimerization with PAR2 or PAR3. The cellular effects of thrombin are mediated by protease-activated receptors (PARs). Thrombin signaling in platelets contributes to hemostasis and thrombosis.

PAR1 is expressed not only in all types of blood cells, but also in epithelium, neurons, astrocytes, and immune cells. PAR1 was originally identified as the thrombin receptor, although other agonists, such as activated protein C (APC), have been identified later. Proteolytic removal of part of the N-terminal extracellular region of PAR1 by these protease agonists results in a newly tethered ligand that interacts with the body of the receptor to induce transmembrane signaling thereby triggering a broad range of signaling pathways. For example, PAR1 participates in the regulation of vascular tone and permeability of endothelial cells. In vascular smooth muscle, it mediates contraction, proliferation, and hypertrophy. PAR1 contributes to the pro-inflammatory response observed in atherosclerosis and restenosis.

Protease activated receptor 3 (PAR3) also known as coagulation factor II receptor-like 2 (F2RL2) and thrombin receptor-like 2, is a protein that in humans is encoded by the F2RL2 gene. PAR3 is activated by proteolytic cleavage of its extracellular amino terminus. Like PAR1, the new amino terminus of PAR3 functions as a tethered ligand and activates the receptor. It is known that PAR3 is a cofactor for PAR4 activation by thrombin in mouse platelets while PAR3 can contribute to PAR1 activation in human cells. Like PAR1, PAR3 is also expressed on vascular endothelial cells. Similarly low level but detectable PAR3 expression is also evident in human platelets.

As used herein, "treating," "treatment" or "ameliorating" refers to (i) preventing a pathologic condition (e.g., an inflammatory disorder) from occurring (e.g. prophylaxis); (ii) inhibiting the pathologic condition or arresting its development; and (iii) relieving symptoms associated with the pathologic condition. Thus, "treatment" includes the administration of the therapeutic compounds or compositions described herein to prevent or delay the onset of the symptoms, complications, or biochemical indicia of a disease described herein, alleviating or ameliorating the symptoms or arresting or inhibiting further development of the disease, condition, or disorder. "Treatment" further refers to any indicia of success in the treatment or amelioration or prevention of the disease, condition, or disorder described herein, including any objective or subjective parameter such as abatement; remission; diminishing of symptoms or making the disease condition more tolerable to the patient; slowing in the rate of degeneration or decline; or making the final point of degeneration less debilitating. Detailed procedures for the treatment or amelioration of a disorder or symptoms thereof can be based on objective or subjective parameters, including the results of an examination by a physician.

As used herein, the term "variant" refers to a molecule (e.g., a polypeptide or polynucleotide) that contains a sequence that is substantially identical to the sequence of a reference molecule. For example, the reference molecule can be an N terminally truncated PAR3 or PAR1 polypeptide (e.g., SEQ ID NO:2 or SEQ ID NO:5) or a polynucleotide encoding the polypeptide. The reference molecule can also be a PAR3 or PAR1 derived anti-inflammatory polypeptide disclosed herein or a polynucleotide encoding the anti-inflammatory polypeptide (e.g., P3R or TR47). In some embodiments, the variant can share at least 50%, at least 70%, at least 80%, at least 90, at least 95% or more sequence identity with the reference molecule. In some other embodiments, the variant differs from the reference molecule by having one or more conservative amino acid substitutions. In some other embodiments, a variant of a reference molecule (e.g., anti-inflammatory polypeptide P3R or TR47) has altered amino acid sequences (e.g., with one or more conservative amino acid substitutions) but substantially retains the biological activity of the reference molecule (e.g., activating PAR3 or PAR1 signaling). Conservative amino acid substitutions are well known to one skilled in the art.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors").

### III. PAR3- and PAR1- derived anti-inflammatory peptides and derivative compounds

The invention provides methods and compositions related to using PAR3- and PAR1-derived anti-inflammatory peptides or polypeptides to suppress inflammation and to treat inflammatory conditions or disorders. PAR3- and PAR1-derived anti-inflammatory peptides respectively refer to peptides or polypeptides that possess anti-inflammatory activities and are derived from or fragments of the N-terminal sequence of PAR3 and PAR1 after cleavage by APC at the non-canonical cleavage site. In various embodiments, the PAR3- and PAR1-derived anti-inflammatory peptides can also possess cytoprotective activities. Cytoprotective activities of the peptides encompass any non-anticoagulant protective cellular activities mediated by the APC signaling pathway, esp. activities related to inhibition of apoptosis and promotion of cell survival. They include activation of the PI3K-Akt survival pathway, prevention of thrombin-induced endothelial barrier disruption, inhibition of endothelial apoptosis (e.g., by blocking the pro-apoptotic activity of p53 or by other mechanisms), secretion of TNF-α by macrophages, reduction of cellular NFκB activation in endothelial cells, prevention of leukocyte adhesion to activated endothelial cells, induction of stabilization of endothelial cell barrier integrity via sphingosine-1 phosphate release or sphingosine-1 phosphate receptor 1 (S1P1) activation or via Tie2 activation. Such activities can be readily assessed with methods that are well known in the art, e.g., endothelial barrier assay and in vivo vascular permeability assay. See, e.g., Mosnier et al., Blood. 120:5237-5246, 2012; Burnier and Mosnier, Blood 122:807-816, 2013; and Stavenuiter and Mosnier, Blood 124:3480-3489, 2014.

As respectively exemplified herein with peptides P3R and TR47 or various fragments thereof, PAR3- and PAR1-derived anti-inflammatory peptides activate PARI and PAR3 differently and cause different kinds of cellular signaling than do peptides that mimic the PAR1 sequence beginning at residue 42, so-called TRAP reagents, or the PAR3 sequence beginning at residue 39, the P3K reagent. Any PAR3- and PAR1-derived anti-inflammatory peptides derived from the N-terminal sequence of PAR3 and PAR1 after cleavage by APC at the non-canonical cleavage site may be employed in the invention. Specifically, PAR1 is cleaved by APC (and other proteases, notably by thrombin) at the canonical site of Arg⁴¹. Peptides that mimic the new N- terminus created when cleavage at Arg⁴¹ are often referred to as Thrombin Receptor Activating Peptides (TRAP). Thrombin cleavage of PAR1 or treatment of cells with TRAP are generally proinflammatory and can often be deleterious to cells or animals, depending on the cell, organ, or context for an animal. APC also cleaves PAR1 at the non-canonical site Arg⁴⁶, resulting in a new N-terminus as shown in Peptide TR47 (aka "P1(47-66)" herein) (47-NPNDKYEPFWEDEEKNESGL-66; SEQ ID NO:7). TRAP and TR47 are agonists for PAR1 activation. However, it has been shown that TR47 acts as an agonist with an activity profile that is distinctly different from that of TRAP. PAR3 is cleaved at a canonical site (Lys38) by proteases such as thrombin. Peptides that mimic the new N-terminus created when cleavage at Lys38 occurs (e.g., a sequence beginning with Thr39 such as peptide "P3K") can act as agonist of PAR3 in activating thrombin mediated pathway. Similar to PAR1, PAR3 can also be cleaved at a non-canonical site (Arg⁴¹), resulting in a new N-terminus as shown in Peptide P3R (aka "P3(42-65)" herein) (42- GAPPNSFEEFPFSALEGWTGATIT-65; SEQ ID NO:4). Peptides P3R and P3K are agonists for PAR3 activation. However, it has been shown that P3R appears to act as an agonist with an activity profile that is distinctly different from that of P3K.

As exemplified herein, PAR1-derived anti-inflammatory peptides mimicking partial sequence of PAR1 that begin with the N-terminal residue Asn⁴⁷ (e.g., peptide TR47) are capable of causing anti-inflammatory cell signaling. The anti-inflammatory cell signaling can be demonstrated by activities such as suppression of the inflammasome, and/or inhibition of caspase 1 activation. Similarly, PAR3-derived anti-inflammatory peptides mimicking partial sequences of PAR3 that begin with the N-terminal residue Gly⁴² (e.g., Peptide P3R), or some fragments thereof, are also capable of causing anti-inflammatory cell signaling. P3R and P3K are agonists for PAR3 activation. Examples show that P3R appears to act as an agonist with an activity profile that is distinctly different from that of P3K.

Typically, PAR3-derived anti-inflammatory peptides for practicing the invention contain (1) at least the first 4 N-terminal residues of Met¹-Arg⁴¹ deleted human PAR3 sequence (SEQ ID NO:2) or conservatively modified variant thereof, or (2) at least 4 contiguous amino acid residues of human PAR3 derived peptide P3R (SEQ ID NO:4) that encompass the Phe¹⁰-Phe¹² motif of SEQ ID NO:3 or conservatively modified variant thereof. In some embodiments, the peptide has a sequence that is derived from the N-terminal residues of the soluble, Met¹-Arg⁴¹ deleted human PAR3 extracellular sequence (SEQ ID NO:3). In some of these embodiments, the anti-inflammatory PAR3-derived polypeptide contains the first 25 or more N-terminal residues of SEQ ID NO:3 or a conservatively modified variant sequence. In some embodiments, the anti-inflammatory PAR3-derived polypeptide contains the first 4, 5, 6, 7, 8, 9, 10, 11, 12 or more N-terminal residues of Peptide P3R (SEQ ID NO:4) or a conservatively modified variant sequence. In some embodiments, the anti-inflammatory PAR3-derived polypeptide contains the first 13, 14, 15, 16, 17, 18, 19, 20, 21, 22, 23 or 24 N-terminal residues of Peptide P3R (SEQ ID NO:4) or a conservatively modified variant sequence. In some embodiments, the anti-inflammatory PAR3-derived polypeptide contains at least 5, 6, 7, 8, 9, 10, 11, 12 or more contiguous amino acid residues of SEQ ID NO:4 that encompass the Phe¹⁰-Phe¹² motif or conservatively modified variant thereof. In some embodiments, the anti-inflammatory PAR3-derived polypeptide contains at least 13, 14, 15, 16, 17, 18, 19, 20, 21, 22, or more contiguous amino acid residues of SEQ ID NO:4 that encompass the Phe¹⁰-Phe¹² motif or conservatively modified variant thereof. Some of these PAR3 derived peptides contain a sequence that encompasses the Phe¹⁰-Trp¹⁸ motif (SEQ ID NO:10) or conservatively modified variant. A few specific examples of the PAR3-derived anti-inflammatory polypeptides suitable for the invention are P3R (SEQ ID NO:4), P3Rm or P3(42-54) (GAPPNSFEEFPFS; SEQ ID NO:8), P3R51-65 or P3(51-65) (FPFSALEGWTGATIT; SEQ ID NO:9), and P3R51-59 or P3(51-59) (FPFSALEGW; SEQ ID NO:10), or conservatively modified variants thereof. Additional examples of PAR3-derived anti-inflammatory polypeptides include GAPPNSFEEFPFSA (SEQ ID NO:11), GAPPNSFEEFPFSAL (SEQ ID NO:12), GAPPNSFEEFPFSALE (SEQ ID NO:13), GAPPNSFEEFPFSALEG (SEQ ID NO:14), GAPPNSFEEFPFSALEGW (SEQ ID NO:15), GAPPNSFEEFPFSALEGWT (SEQ ID NO: 16), GAPPNSFEEFPFSALEGWTG (SEQ ID NO:17), GAPPNSFEEFPFSALEGWTGA (SEQ ID NO:18), GAPPNSFEEFPFSALEGWTGAT (SEQ ID NO:19), GAPPNSFEEFPFSALEGWTGATI (SEQ ID NO:20), FPFSALEGWT (SEQ ID NO:21), FPFSALEGWTG (SEQ ID NO:22), FPFSALEGWTGA (SEQ ID NO:23), FPFSALEGWTGAT (SEQ ID NO:24), FPFSALEGWTGATI (SEQ ID NO:25), FPFSALEG (SEQ ID NO:26), FPFSALE (SEQ ID NO:27), FPFSAL (SEQ ID NO:28), EFPFSAL (SEQ ID NO:29), and EEFPFSAL (SEQ ID NO:30), or conservatively modified variants thereof. In the methods of the invention for suppressing inflammation and/or for treating inflammatory conditions, any of these PAR3-derived peptides can be used alone or in combination with a PAR1-derived peptide described herein.

Typically, PAR1-derived anti-inflammatory peptides for practicing the invention have at least the first 4 or 5 N-terminal residues that are substantially identical to the corresponding N-terminal residues of Met¹-Arg⁴⁶ deleted human PAR1 sequence (SEQ ID NO:5), variants (e.g., Met¹-Arg⁴⁶ deleted human PAR1 sequence with conservative substitutions), orthologs (e.g., non-human PAR1 sequences with similar deletions), or are TR47 (aka P1(47-66)) (SEQ ID NO:7) variants with further N-terminal modifications (amino acid substitution or deletions). Preferably, the peptide has a sequence that is derived from the N-terminal residues of the soluble, Met¹-Arg⁴⁶ deleted human PAR1 extracellular sequence (SEQ ID NO:6). In some embodiments, the anti-inflammatory PAR1-derived polypeptide has at least the first 4 N-terminal residues that are substantially identical to the corresponding N-terminal residues of SEQ ID NO:6. In some of these embodiments, the anti-inflammatory PAR1-derived polypeptide contains the first 21 or more N-terminal residues of SEQ ID NO:6 or a conservatively modified variant sequence. In some preferred embodiments, the PAR1 derived anti-inflammatory peptide contains the first 6, 7, 8, 9, 10, 11, 12 or more N-terminal residues of Peptide TR47 (SEQ ID NO:7) or a conservatively modified variant sequence. As exemplified herein with TR47 8-mer, TR47 9-mer and TR47 16-mer, these peptides maintain substantive anti-inflammatory activities relative to that of TR47. In some embodiments, the PAR1 derived anti-inflammatory peptide contains the first 13, 14, 15, 16, 17, 18, 19 or more N-terminal residues of SEQ ID NO:7 or a conservatively modified variant sequence. In some other embodiments, the PAR1 derived anti-inflammatory peptide is a variant of TR47 with mutations at the N-terminus. These include TR47 variants with deletions of the first 1, 2, 3, 4, 5, 6, 7, 8 or more residues at the N-terminus. They also include TR47 variants with one or more amino acid substitutions at the N-terminus. As exemplified herein with TR47(N-1), TR47(N-5), TR47ΔQ and TR47ΔA, these TR47 variant peptides also maintain substantial anti-inflammatory activities relative to that of TR47.

Some specific examples of the PAR1-derived anti-inflammatory polypeptides suitable for the invention are shown in SEQ ID NO:6 and SEQ ID NO:7 (Peptide TR47), and also peptides NPND (SEQ ID NO:31), NPNDK (SEQ ID NO:32), NPNDKY (SEQ ID NO:33), NPNDKYE (SEQ ID NO:34), NPNDKYEP (SEQ ID NO:35), NPNDKYEPF (SEQ ID NO:36), NPNDKYEPFW (SEQ ID NO:37), NPNDKYEPFWE (SEQ ID NO:38), NPNDKYEPFWED (SEQ ID NO:39), NPNDKYEPFWEDE (SEQ ID NO:40), NPNDKYEPFWEDEE (SEQ ID NO:41), NPNDKYEPFWEDEEK (SEQ ID NO:42), NPNDKYEPFWEDEEKN (SEQ ID NO:43), NPNDKYEPFWEDEEKNE (SEQ ID NO:44), NPNDKYEPFWEDEEKNES (SEQ ID NO:45), NPNDKYEPFWEDEEKNESG (SEQ ID NO:46), PNDKYEPFWEDEEKNESGL (SEQ ID NO:47), NDKYEPFWEDEEKNESGL (SEQ ID NO:51), DKYEPFWEDEEKNESGL (SEQ ID NO:57), KYEPFWEDEEKNESGL (SEQ ID NO:58), YEPFWEDEEKNESGL (SEQ ID NO:48), EPFWEDEEKNESGL (SEQ ID NO:59), PFWEDEEKNESGL (SEQ ID NO:60), QPNDKYEPFWEDEEKNESGL (SEQ ID NO:49) and APNDKYEPFWEDEEKNESGL (SEQ ID NO:50). In the methods of the invention for suppressing inflammation and/or for treating inflammatory conditions, any of these PAR1-derived peptides can be used alone or in combination with a PAR3-derived peptide described herein.

In addition to the peptides noted above, anti-inflammatory compounds suitable for the invention also include related or derivative compounds that are derived from the PAR3- and PAR1-derived anti-inflammatory peptides or polypeptides exemplified herein. These related or derivative compounds encompass variants, analogs, mimetics, complexes or fusion molecules containing the peptides or polypeptides, and other compounds derived from the PAR3- and PAR1-derived anti-inflammatory peptides or polypeptides. In some preferred embodiments, the derivative compounds possess substantially the same chemical or biological activities as the PAR3- and PAR1-derived anti-inflammatory peptides or polypeptides exemplified herein (e.g., P3R and TR47 peptides). The related or derivative compounds can include, e.g., peptides or polypeptides that have substantially identical sequences, e.g., conservatively modified variants. They also include peptides or polypeptides that are derived from non-human ortholog PAR3 and PAR1 sequences, respectively. Related or derivative compounds suitable for the invention also encompass variants, analogs, multimers, peptidomimetics, fusions with other molecules (e.g., carrier moieties) or other derivatives that can be generated from the PAR3- and PAR1 - derived anti-inflammatory polypeptides exemplified herein (e.g., Peptide P3R and TR47). These derivative compounds can be subject to the appropriate assays or screening methods to identify anti-inflammatory compounds with optimized activities. In some embodiments, the derivative compounds are modified versions of the exemplified peptides which are generated by conservative amino acid substitutions. In some other embodiments, the derivative compounds are variants produced by non-conservative substitutions to the extent that that they substantially retain the activities of those peptides. Modification to an anti-inflammatory peptide can be performed with standard techniques routinely practiced in the art (e.g., U.S. Patent Applications 20080090760 and 20060286636).

In some embodiments, the derivative compounds of an exemplified PAR-3 and PAR1-derived anti-inflammatory polypeptide of the invention (e.g., peptide P3R or TR47) are analogs or that contain one or more naturally occurring amino acid derivatives of the twenty standard amino acids, for example, 4-hydroxyproline, 5-hydroxylysine, 3-methylhistidine, homoserine, ornithine or carboxyglutamate, and can include amino acids that are not linked by polypeptide bonds. Similarly, they can also be cyclic polypeptides and other conformationally constrained structures. Methods for modifying a polypeptide to generate analogs and derivatives are well known in the art, e.g., Roberts and Vellaccio, The Peptides: Analysis, Synthesis, Biology, Eds. Gross and Meinhofer, Vol. 5, p. 341, Academic Press, Inc., New York, N.Y. (1983); and Burger's Medicinal Chemistry and Drug Discovery, Ed. Manfred E. Wolff, Ch. 15, pp. 619-620, John Wiley & Sons Inc., New York, N.Y. (1995).

Some other derivative compounds of the exemplified PAR3- and PAR1-derived anti-inflammatory polypeptides are peptidomimetics. Peptidomimetics based on a PAR3- and PAR1-derived anti-inflammatory peptide (e.g., peptide P3R or TR47) substantially retain the activities of the reference peptide. They include chemically modified peptides or polypeptides, polypeptide-like molecules containing non-naturally occurring amino acids, peptoids and the like, that have a structure substantially the same as the reference polypeptide or peptide upon which the peptidomimetic is derived (see, for example, Burger's Medicinal Chemistry and Drug Discovery, 1995, supra). For example, the peptidomimetics can have one or more residues chemically derivatized by reaction of a functional side group. In addition to side group derivatizations, a chemical derivative can have one or more backbone modifications including alpha-amino substitutions such as N-methyl, N-ethyl, N-propyl and the like, and alpha-carbonyl substitutions such as thioester, thioamide, guanidino and the like. Typically, a peptidomimetic shows a considerable degree of structural identity when compared to the reference polypeptide and exhibits characteristics which are recognizable or known as being derived from or related to the reference polypeptide. Peptidomimetics include, for example, organic structures which exhibit similar properties such as charge and charge spacing characteristics of the reference polypeptide. Peptidomimetics also can include constrained structures so as to maintain optimal spacing and charge interactions of the amino acid functional groups.

In some embodiments, derivative compounds of PAR3- and PAR1-derived anti-inflammatory peptides or polypeptides described herein are molecules that have the peptides or polypeptides covalently or non-covalently conjugated to a carrier moiety via any conventional methods. A "carrier moiety" ("carrier" or "carrier molecule") is a conjugation partner capable of enhancing the immunogenicity of a polypeptide. Suitable carriers are typically large, slowly metabolized macromolecules such as proteins; polysaccharides (such as latex functionalized SEPHAROSE^{™}, agarose, cellulose, cellulose beads and the like); polymeric amino acids (such as polyglutamic acid, polylysine, and the like); amino acid copolymers; and inactive virus particles or attenuated bacteria, such as Salmonella. In various embodiments, the carrier moiety can be a carrier protein, an immunoglobulin, a Fc domain, a PEG molecule or other polymer. In some embodiments, the carrier moiety is a protein. Integral membrane proteins from, e.g., *E. coli* and other bacteria are useful conjugation partners. Especially useful carrier proteins are serum albumins, keyhole limpet hemocyanin (KLH), certain immunoglobulin molecules, thyroglobulin, ovalbumin, bovine serum albumin (BSA), tetanus toxoid (TT), and diphtheria toxoid (CRM). In some other embodiments, the carrier moiety can be a polymer other than a protein or polypeptide. Examples of such polymers include, e.g., carbohydrates such as dextran, mannose or mannan.

In some embodiments, the PAR3- and PAR1-derived anti-inflammatory peptides, variants or derivatives described herein are dimerized or multimerized molecules. In some of these embodiments, dimerization or multimerization of the anti-inflammatory agents can be achieved by covalent attachment to at least one linker moiety. In some embodiments for combination therapy, a PAR3-derived peptide or variant (e.g., P3R or P3R (51-59)) can be conjugated to a PAR1-derived peptide or variant (e.g., TR47 or TR47 8-mer). Either homologous multimerization or heterologous multimerization of PAR3- and/or PAR1-derived peptides can be achieved via various suitable means. For example, the peptides can be covalently linked via recombinant techniques. In some embodiments, the multimerized molecules of the invention can utilize a carrier moiety noted above. In some of these embodiments, a carrier molecule or moiety can be used to conjugate a PAR3-derived peptide (e.g., P3R or P3R (51-59)) to a PAR1-derived peptide (e.g., TR47 or TR47 8-mer). In some embodiments, the peptides or polypeptides can also be conjugated with a C₁₋₁₂ linking moiety optionally terminated with one or two -NH- linkages and optionally substituted at one or more available carbon atoms with a lower alkyl substituent.

The PAR3 and/or PAR1 derived peptides described herein can be joined by other chemical bond linkages, such as linkages by disulfide bonds or by chemical bridges. In some other embodiments, the anti-inflammatory peptides described herein can be linked physically in tandem to form a polymer of PAR3- or PAR1-derived peptides. The peptides making up such a polymer can be spaced apart from each other by a peptide linker. For example, a PAR3 derived peptide can be covalently bonded to a PAR1 derived peptide via a glycine linker as exemplified herein. In various embodiments, the glycine linker can contain from about 2 to about 20 glycine residues. In these embodiments, the PAR3 derived peptide can be bonded via the linker to either the N-terminus or the C-terminus of the PAR1 derived peptide. In some embodiments, other molecular biology techniques well known in the art can be used to create a polymer of peptides. In some embodiments, polyethylene glycol (PEG) may serve as a linker that dimerizes two peptide monomers. For example, a single PEG moiety containing two reactive functional groups may be simultaneously attached to the N-termini of both peptide chains of a peptide dimer. These peptides are referred to herein as "PEGylated peptides." In some embodiments, the peptide monomers of the invention may be oligomerized using the biotin/streptavidin system.

In any of these conjugation schemes, varying copies of the PAR3-derived peptide (e.g., P3R or P3R (51-59)) and the PAR1-derived peptide (e.g., P3R or P3R (51-59)) can be linked in the heterologously multimerized molecule. In some of these embodiments, multiple copies of the PAR3-derived peptide are attached to the carrier moiety for each copy of the attached PAR1-derived peptide. For example, the multimerized molecule can contain a carrier moiety with the PAR3-derived peptide and the PAR1-derived peptide attached at a ratio of at least about 20:1, 15:1, 12:1, 10:1, 8:1, 6:1, 4:1, or 2:1. In some other embodiments, multiple copies of the PAR1-derived peptide are attached to the carrier moiety for each copy of the attached PAR3-derived peptide. For example, the multimerized molecule can contain a carrier moiety with the PAR1-derived peptide and the PAR3-derived peptide attached at a ratio of at least about 20:1, 15:1, 12:1, 10:1, 8:1, 6:1, 4:1, or 2:1. Depending on the specific dosages to be achieved for the two peptides or their derivative compounds as described herein, any of these conjugation ratios can be used in constructing the heterologously multimerized molecule for practicing the methods of the invention.

Methods for stabilizing peptides known in the art may be used with the methods and compositions described herein. For example, using D-amino acids, using reduced amide bonds for the peptide backbone, and using non-peptide bonds to link the side chains, including, but not limited to, pyrrolinone and sugar mimetics can each provide stabilization. The design and synthesis of sugar scaffold peptide mimetics are described in the art, e.g., Hirschmann et al., J. Med. Chem. 36, 2441-2448, 1996. Further, pyrrolinone-based peptide mimetics present the peptide pharmacophore on a stable background that has improved bioavailability characteristics. See, e.g., Smith et al., J. Am. Chem. Soc. 122, 11037-11038, 2000.

In some embodiment, derivative compounds of the exemplified anti-inflammatory PAR3 and PAR1 peptides or polypeptides include modifications within the sequence, such as, modification by terminal-NH₂ acylation, e.g., acetylation, or thioglycolic acid amidation, by terminal-carboxylamidation, e.g., with ammonia, methylamine, and the like terminal modifications. One can also modify the amino and/or carboxy termini of the polypeptides described herein. Terminal modifications are useful to reduce susceptibility by proteinase digestion, and therefore can serve to prolong half-life of the polypeptides in solution, particularly in biological fluids where proteases may be present. Amino terminus modifications include methylation (e.g., -NHCH₃ or -N(CH₃)₂), acetylation (e.g., with acetic acid or a halogenated derivative thereof such as α-chloroacetic acid, α-bromoacetic acid, or α-iodoacetic acid), adding a benzyloxycarbonyl (Cbz) group, or blocking the amino terminus with any blocking group containing a carboxylate functionality defined by RCOO- or sulfonyl functionality defined by R-SO₂-, where R is selected from the group consisting of alkyl, aryl, heteroaryl, alkyl aryl, and the like, and similar groups. One can also incorporate a desamino acid at the N-terminus (so that there is no N-terminal amino group) to decrease susceptibility to proteases or to restrict the conformation of the peptide compound. In some embodiments, the N-terminus is acetylated with acetic acid or acetic anhydride.

Carboxy terminus modifications include replacing the free acid with a carboxamide group or forming a cyclic lactam at the carboxy terminus to introduce structural constraints. One can also cyclize the peptides described herein, or incorporate a desamino or descarboxy residue at the termini of the peptide, so that there is no terminal amino or carboxyl group, to decrease susceptibility to proteases or to restrict the conformation of the peptide. Methods of circular peptide synthesis are known in the art, for example, in U.S. Patent Application No. 20090035814; and Muralidharan and Muir, Nat. Methods, 3:429-38, 2006. C-terminal functional groups of the peptides described herein include amide, amide lower alkyl, amide di(lower alkyl), lower alkoxy, hydroxy, and carboxy, and the lower ester derivatives thereof, and the pharmaceutically acceptable salts thereof.

The PAR3- and PAR1- derived anti-inflammatory peptide or polypeptide compounds described herein also serve as structural models for non-peptidic compounds with similar biological activity. There are a variety of techniques available for constructing compounds with the same or similar desired biological activity as the PAR3- and PAR1-derived peptides, but with more favorable activity with respect to solubility, stability, and susceptibility to hydrolysis and proteolysis. See, e.g., Morgan and Gainor, Ann. Rep. Med. Chem. 24:243-252, 1989. These techniques include, but are not limited to, replacing the peptide backbone with a backbone composed of phosphonates, amidates, carbamates, sulfonamides, secondary amines, and N-methylamino acids.

### IV. Synthesis of PAR3- and PAR1- derived anti-inflammatory peptides and related compounds

The PAR3- and PAR1- derived anti-inflammatory polypeptides described herein, including variants and derivatives thereof, can be chemically synthesized and purified by standard chemical or biochemical methods that are well known in the art. Some of the methods for generating analog or derivative compounds of the PAR3- and PAR1- derived anti-inflammatory polypeptides are described above. Other methods that may be employed for producing the anti-inflammatory polypeptides of the invention and their derivative compounds include, e.g., solid phase peptide synthesis. For example, the peptides can be synthesized using t-Boc (tert-butyloxycarbonyl) or FMOC (9-flourenylmethloxycarbonyl) protection group described in the art. See, e.g., Merrifield, J. Am. Chem. Soc. 85:2149-2154, 1963; "Peptide synthesis and applications" in Methods in molecular biology Vol. 298, Ed. by John Howl; "Chemistry of Peptide Synthesis" by N. Leo Benoiton, 2005, CRC Press, (ISBN-13: 978-1574444544); and "Chemical Approaches to the Synthesis of Peptides and Proteins" by P. Lloyd-Williams, et. al., 1997, CRC-Press, (ISBN-13: 978-0849391422), Methods in Enzymology, Volume 289: Solid-Phase Peptide Synthesis, J. N. Abelson, M. I. Simon, G. B. Fields (Editors), Academic Press; 1st edition (1997) (ISBN-13: 978-0121821906); U.S. Pat. Nos. 4,965,343, and 5,849,954.

In some embodiments, the peptides can be obtained via solid phase synthesis using peptide synthesizing machines. Commercial peptide synthesizing machines are available for solid phase peptide synthesis. For example, the Advanced Chemtech Model 396 Multiple Peptide Synthesizer and an Applied Biosystems Model 432A Peptide synthesizer are suitable. There are commercial companies that make custom synthetic peptides to order, e.g., Abbiotec, Abgent, AnaSpec Global Peptide Services, LLC., Invitrogen, and rPeptide, LLC.

In some embodiments, the PAR3- and PAR1 derived- anti-inflammatory polypeptides and derivatives thereof can also be synthesized and purified by molecular methods that are well known in the art. Recombinant polypeptides may be expressed in bacteria, mammal, insect, yeast, or plant cells. For example, conventional polymerase chain reaction (PCR) cloning techniques can be used to clone a polynucleotide encoding a PAR3-derived or a PAR1-derived peptide or polypeptide, using the full length PAR3 or PAR1 cDNA sequence as the template for PCR Cloning. Alternatively, the sense and anti-sense strand of the coding nucleic acid can be made synthetically and then annealed together to form the double-stranded coding nucleic acid. Ideally, restriction enzyme digestion recognition sites should be designed at the ends of the sense and anti-sense strand to facilitate ligation into a cloning vector or other vectors. Alternatively, a 3'A- overhang can be include for the purpose of TA-cloning that is well known in the art. Such coding nucleic acids with 3'A- overhangs can be easily ligated into the Invitrogen topoisomerase-assisted TA vectors such as pCR-TOPO, pCR-Blunt II-TOPO, pENTR/D-TOPO, and pENTR/SD/D-TOPO. The coding nucleic acid can be cloned into a general purpose cloning vector such as pUC19, pBR322, pBluescript vectors (Stratagene Inc.) or pCR-TOPO from Invitrogen Inc. The resultant recombinant vector carrying the polynucleotide encoding a PAR3 or PAR1 peptide can then be used for further molecular biological manipulations. These include, e.g., site-directed mutagenesis for variant PAR1 peptide and/or to reduce the immunogenic properties of the peptide or improve protein expression in heterologous expression systems. The coding sequences can also be subcloned into protein expression vectors or viral vectors for the synthesis of fusion protein comprising PAR1 peptides and protein synthesis in a variety of protein expression systems. These include expression systems based on host cells selected from the group consisting of mammalian cell lines, insect cell lines, yeast, bacteria, and plant cells.

In some related embodiments, the invention provides isolated or substantially purified polynucleotides (DNA or RNA) which encode the PAR3- and PAR1-derived anti-inflammatory polypeptides described herein, including heterologous multimers (e.g., dimers) containing both a PAR3-derived peptide and a PAR1-derived peptide. Expression vectors and engineered host cells harboring the vectors for expressing polynucleotides encoding the polypeptides are also provided in the invention. The polynucleotide encoding the anti-inflammatory polypeptide (e.g., a multimer of P3R (51-59) /TR47 8-mer) are operationally linked to a promoter in the expression vectors. The expression construct can further comprise a secretory sequence to assist purification of the peptide from the cell culture medium. The host cells to which the vectors are introduced can be any of a variety of expression host cells well known in the art, e.g., bacteria (e.g., E. coli), yeast cell, or mammalian cells.

Recombinant protein expression in different host cells can be constitutive or inducible with inducers such as copper sulfate, or sugars such as galactose, methanol, methylamine, thiamine, tetracycline, or IPTG. After the protein is expressed in the host cells, the host cells are lysed to liberate the expressed protein for purification. A preferred purification method is affinity chromatography such as ion-metal affinity chromatograph using nickel, cobalt, or zinc affinity resins for histidine-tagged peptide. Methods of purifying histidine-tagged recombinant proteins are described by Clontech using their Talon^{®} cobalt resin and by Novagen in their pET system manual, 10th edition. Another preferred purification strategy is by immuno-affinity chromatography, for example, anti-Myc antibody conjugated resin can be used to affinity purify Myc-tagged peptide. Enzymatic digestion with serine proteases such as thrombin and enterokinase cleave and release the peptide from the histidine or Myc tag, releasing the recombinant peptide from the affinity resin while the histidine-tags and Myc-tags are left attached to the affinity resin.

Cell-free expression systems can also be used for producing anti-inflammatory polypeptides of the invention. Cell-free expression systems offer several advantages over traditional cell-based expression methods, including the easy modification of reaction conditions to favor protein folding, decreased sensitivity to product toxicity and suitability for high-throughput strategies such as rapid expression screening or large amount protein production because of reduced reaction volumes and process time. The cell-free expression system can use plasmid or linear DNA. Moreover, improvements in translation efficiency have resulted in yields that exceed a milligram of protein per milliliter of reaction mix. An example of a cell-free translation system capable of producing proteins in high yield is described by Spirin et. al., Science 242:1162, 1988. The method uses a continuous flow design of the feeding buffer which contains amino acids, adenosine triphosphate (ATP), and guanosine triphosphate (GTP) throughout the reaction mixture and a continuous removal of the translated polypeptide product. The system uses E. coli lysate to provide the cell-free continuous feeding buffer. This continuous flow system is compatible with both prokaryotic and eukaryotic expression vectors. An example of large scale cell-free protein production is described in Chang et. al., Science 310:1950-3, 2005.

Other commercially available cell-free expression systems include the Expressway^{™} Cell-Free Expression Systems (Invitrogen) which utilize an E. coli-based in-vitro system for efficient, coupled transcription and translation reactions to produce up to milligram quantities of active recombinant protein in a tube reaction format; the Rapid Translation System (RTS) (Roche Applied Science) which also uses an E. coli-based in-vitro system; and the TNT Coupled Reticulocyte Lysate Systems (Promega) which uses a rabbit reticulocyte-based in-vitro system.

### V. Suppressing inflammation and treating inflammatory conditions

The anti-inflammatory PAR3- and PAR1- derived anti-inflammatory peptides and derivative compounds described herein, including variants, analogs, and peptidomimetics, can be employed in many therapeutic or prophylactic applications to inhibit or suppress undesired inflammation, and to treat many inflammatory diseases or disorders. In some embodiments, a subject in need of treatment is administered a therapeutically effective amount of a PAR3-derived anti-inflammatory peptide or polypeptide or derivative compound described herein. In some embodiments, a subject in need of treatment is administered a therapeutically effective amount of a PAR1-derived anti-inflammatory peptide or polypeptide or derivative compound described herein. In some other embodiments, a subject in need of treatment is administered both a PAR3-derived anti-inflammatory peptide or polypeptide or derivative compound and a PAR3-derived anti-inflammatory peptide or polypeptide or derivative compound as described herein. As detailed herein, the combination of both compounds, even at very low dosages, can achieve synergistic effect in suppressing or inhibiting inflammation. As detailed below, the anti-inflammatory peptides (e.g., P3R or TR47), with or without homologous or heterologous multimerization (conjugation or fusion), can be formulated in pharmaceutical compositions for the therapeutic or prophylactic applications disclosed herein.

In one aspect, the invention provides methods for suppressing inflammation and treating subjects suffering from inflammatory diseases. The PAR3- and PAR1-derived anti-inflammatory peptides, variants and mimetics described herein can be employed for suppressing or ameliorating symptoms associated with undesired inflammation, and for the treatment of various diseases or disorders that are associated with or mediated by undesired inflammatory responses. These include conditions characterized or associated with undesired immune activation or undesired immune responses, e.g., allogeneic transplants rejections, autoimmune diseases (e.g., lupus and multiple sclerosis), allergic reactions (e.g., asthma), inflammatory conditions in the epidermis (eczema), rheumatoid arthritis, sterile inflammation, and hay fever. Specific diseases or disorders include, e.g., graft vs. host diseases, rheumatoid arthritis, systemic lupus erythematosus, Hashimoto's thyroidis, multiple sclerosis, myasthenia gravis, neuromyelitis optica (NMO), diabetes type I or II and the disorders associated therewith, vasculitis, pernicious anemia, Sjogren's syndrome, uveitis, psoriasis, Graves ophthalmopathy, alopecia areata and others, allergic diseases, e.g. allergic asthma, atopic dermatitis, allergic rhinitis/conjunctivitis, allergic contact dermatitis, inflammatory diseases optionally with underlying aberrant reactions, e.g. inflammatory bowel disease, Crohn's disease or ulcerative colitis, intrinsic asthma, inflammatory lung injury, inflammatory liver injury, inflammatory glomerular injury, atherosclerosis, osteoarthritis, irritant contact dermatitis and further eczematous dermatitises, seborrhoeic dermatitis, cutaneous manifestations of immunologically-mediated disorders, inflammatory eye disease, keratoconjunctivitis, and acute respiratory distress syndrome. The conditions characterized or associated with undesired immune activation or undesired immune responses also include viral infections, including infections by, e.g., chikungunya virus, HIV-1, coronaviruses, and flu viruses.

In various embodiments, the methods of the invention are directed to treatment of inflammatory disorders and infectious diseases. Inflammatory disorders that may be suitable for the prognostic methods of the invention include any diseases or conditions associated with abnormal inflammation and in particular disorders that are mediated by or associated with Type I IFN amplification pathway and cytokine storm. These include, e.g., asthma, autoimmune diseases, chronic inflammation, chronic prostatitis, glomerulonephritis, hypersensitivities, inflammatory bowel diseases, pelvic inflammatory diseases, reperfusion injury, rheumatoid arthritis, transplant rejection and vasculitis. Examples of specific inflammatory disorders include, but are not limited to, rheumatoid arthritis, systemic lupus erythematosus, acute respiratory distress syndrome (ARDS), alopecia areata, anklosing spondylitis, antiphospholipid syndrome, autoimmune Addison's disease, autoimmune hemolytic anemia, autoimmune hepatitis, autoimmune inner ear disease, autoimmune lymphoproliferative syndrome (alps), autoimmune thrombocytopenic purpura (ATP), Blehcet's disease, bullous pemphigoid, cardiomyopathy, celiac sprue-dermatitis, chronic fatigue syndrome immune deficiency syndrome (CFIDS), chronic inflammatory demyelinating polyneuropathy, cicatricial pemphigoid, cold agglutinin disease, Crest syndrome, Crohn's disease, Dego's disease, dermatomyositis, dermatomyositis-juvenile, discoid lupus, essential mixed cryoglobulinemia, fibromyalgia-fibromyositis, grave's disease, guillain-barre, hashimoto's thyroiditis, idiopathic pulmonary fibrosis, idiopathic thrombocytopenia purpura (ITP), IgA nephropathy, insulin dependent diabetes (Type 1), juvenile arthritis, Meniere's disease, mixed connective tissue disease, multiple sclerosis, myasthenia gravis, pemphigus vulgaris, pernicious anemia, polyarteritis nodosa, polychondritis, polyglancular syndromes, polymyalgia rheumatica, polymyositis and dermatomyositis, primary agammaglobulinemia, primary biliary cirrhosis, psoriasis, Raynaud's phenomenon, Reiter's syndrome, rheumatic fever, sarcoidosis, scleroderma, sepsis, Sjogren's syndrome, stiff-man syndrome, systemic inflammatory response syndrome (SIRS), Takayasu arteritis, temporal arteritis/giant cell arteritis, ulcerative colitis, uveitis, vasculitis, vitiligo, and Wegener's granulomatosis.

In some embodiments, the undesired inflammation or inflammatory condition to be treated are associated with, mediated or manifested by caspase 1 activation. In some embodiments, the undesired inflammation or inflammatory condition to be treated are associated with, mediated or manifested by inflammasome formation. In some embodiments, the subject to be treated is one who is afflicted with or suspected to have a condition or disorder that has been shown to be amenable to treatment with APC. These include, e.g., ischemia/reperfusion in brain, heart, and kidney; pulmonary, kidney, and gastrointestinal inflammation; sepsis; Ebola virus; diabetes; and total lethal body radiation. See, e.g., Griffin et al., Blood 125:2898-2907, 2015.

In some embodiments, the therapeutic methods of the invention involve administering to a subject a pharmaceutical composition that contains a PAR3 derived anti-inflammatory peptide described herein, e.g., P3R. In some embodiments, the methods entail administering to a subject a pharmaceutical composition that contains a PAR1 derived anti-inflammatory peptide described herein, e.g., TR47. In some other embodiments, the subject in need of treatment is administered with a combination of a PAR3 derived anti-inflammatory peptide described herein and a PAR1 derived anti-inflammatory peptide described herein. In some of these embodiments, the PAR3 derived anti-inflammatory peptide and the PAR1 derived anti-inflammatory peptide are administered to the subject simultaneously. In some other embodiments, the two peptides can be administered to the subject sequentially. In various embodiments, the two peptides can be each independently conjugated to a carrier moiety as described above. In some embodiments, the simultaneously administered PAR3- and PAR1- derived peptides are conjugated to each other, e.g., via a covalent peptide fusion. In these embodiments, the PAR3-derived peptide can be fused at either the N-terminus or the C-terminus of the PAR1-derived peptide. In some of these embodiments, the two peptides can be connected via a linker moiety, e.g., a linker or spacer peptide. In some embodiments, the two peptides with varying ratios can be conjugated to a carrier moiety, e.g., a carrier protein, a Fc domain, or a PEG molecule described above.

Preferably, therapeutic methods of the invention are directed to treating mammalian subjects. In some of these embodiments, the subject is a human patient. Some embodiments of the invention are directed to inhibiting or suppressing undesired inflammation or inflammatory responses that underline or are associated with any of the inflammatory diseases or disorders described herein. Some embodiments of the invention are directed to treating human subjects afflicted with or suspected of having autoimmune disorders. Examples of autoimmune disorders and related diseases suitable for the methods of the invention include, e.g., Acute disseminated encephalomyelitis (ADEM), Addison's disease, Alopecia areata, Ankylosing spondylitis, Antiphospholipid antibody syndrome (APS), Autoimmune hemolytic anemia, Autoimmune hepatitis, Autoimmune inner ear disease, Bullous pemphigoid, Behcet's disease, Coeliac disease, Chagas disease, Chronic obstructive pulmonary disease, Crohn's Disease, Dermatomyositis, Diabetes mellitus type 1, Diabetes-related inflammation, Endometriosis, Goodpasture's syndrome, Graves' disease, graft versus host disease (GVHD), Guillain-Barré syndrome (GBS), Hashimoto's disease, Hidradenitis suppurativa, Kawasaki disease, Idiopathic thrombocytopenic purpura, Interstitial cystitis, Lupus erythematosus, Mixed Connective Tissue Disease, Morphea, Multiple sclerosis (MS), Myasthenia gravis, Narcolepsy, Neuromyotonia, Pemphigus vulgaris, Pernicious anemia, Psoriasis, Psoriatic Arthritis, Polymyositis, Primary biliary cirrhosis, Rheumatoid arthritis, Schizophrenia, Scleroderma, Sjogren's syndrome, Stiff person syndrome, Temporal arteritis (aka "giant cell arteritis"), Ulcerative Colitis, Vasculitis, Vitiligo, Microscopic polyangiitis, Glomerulonephritis, and Wegener's granulomatosis.

In some embodiments, the subject to be treated with methods of the invention is one who is suffering from or suspected of having neuroinflammation. Subjects with either acute neuroinflammation or chronic neuroinflammation are suitable for treatment. It is known that neuroinflammation contributes to acute and chronic neuropathologies. These include stroke, post-traumatic brain injury, Alzheimer's disease, amyotrophic lateral sclerosis, Parkinson's disease, Huntington's disease, frontal temporal dementia, dementia with Lewy bodies, cerebral malaria, and prion disease. Other central nervous system pathologies with excessive inflammation include CNS infectious diseases, such as Zika, HIV, and West Nile virus, coronavirus infection, and bacterial infections that induce meningitis. See, e.g., Voet et al, EMBO Mol. Med. 11:e10248, 2019; Heneka et al., Nat. Rev. Neurosci. 19:610-621, 2018; Ismael et al., Sci. Rep. 8:5971, 2018. Treatment with methods of the invention will be beneficial to cure or ameliorate symptoms of various neuropathologies that are associated with neuroinflammation. By substantially suppressing or inhibiting the underlying inflammation in the listed conditions, subjects suffering from any of these neuropathologies can derive significant benefit from treatment with methods of the invention. In some other embodiments, the subjects to be treated with methods of the invention are ones who are afflicted with or at risk of developing malarial inflammation. Malaria, which is caused by Plasmodium parasite erythrocyte infection, is a highly inflammatory disease with characteristic periodic fevers caused by the synchronous rupture of infected erythrocytes to release daughter parasites. By substantially suppressing or inhibiting the underlying inflammation, subjects suffering from malaria can derive great benefit from treatment with methods of the invention.

### VI. Screening methods for identifying anti-inflammatory agents

The PAR3- and/or PAR1-derived anti-inflammatory peptides and in vitro assays described herein can be used to identify novel agents with anti-inflammatory activities. In some embodiments, the invention provides methods of using the exemplified peptides (e.g., P3R and/or TR47) as positive controls to screen candidate agents for anti-inflammatory activities. Any known screening methods or platform for identifying anti-inflammatory agents can be adapted to incorporate the anti-inflammatory peptides described herein as positive controls. These include any animal models or other in vivo platform, ex vivo screening methods and also in vitro screening methods. Such methods are described in the art, e.g., del Palacio et al., J. Biomole. Screening 21:567-578, 2016; Jiang et al., PLoS One. 9: e96214, 2014; Hall et al., Disease Models & Mechanisms 7:1069-1081, 2014; Phanse et al., J. App. Pharm. Sci. 2:19-33, 2012; Giddings and Maitra, J. Biomole. Screening 15: 1204-1210, 2010; Singh et al., Clinical Chemistry 51:2252-2256, 2005; Maurer et al., J. Anal. Toxicol. 25:237-44, 2001; Alener & Bingoul, Intl. J. Crude Drug Res. 26: 197-207, 1988; Famaey and Whitehouse, Biochem. Pharmacol. 24:1609-1616, 1975; VanArman, Clin. Pharmacol. Ther. 16:900-904, 1974; Carrano et al., J. Pharm. Sci. 61: 1450-1454, 1972; Riesterer et al., Agents and Action 2, 27-32, 1971; Winter et al., Fed. Proc. 23, 284, 1964; and Winter and Portar, J. Amer. Pharm. Sci. Ed. 46:515-519, 1957.

Some screening methods of the invention involve the use of in vitro cultured cell line to monitor an inflammation related activity. In some of these embodiments, the inflammation related activity to be monitored relates to assembly and activation of Inflammasome and Inflammasome-dependent activation of Caspase 1, e.g., enzymatic activity of caspase 1 or secretion of IL-1β. Inflammasome is a large, intracellular multiprotein complex that plays an integral role in inflammation-driven disease states. The Inflammasome can involve the activation of caspase 1, which is known to catalyze the maturation and processing of interleukin (IL)-1β and pro-IL-1β. Neutralization of IL-1β by a monoclonal antibody in the Canakinumab for Atherosclerotic Disease (CANTOS) trial (see, e.g., Ridker et al., N. Engl. J. Med. 377:1119-1131, 2017) has demonstrated that reduction of inflammation without concomitant change in lipid levels reduces the risk of atherothrombosis, and is strong evidence pointing to the broad reach of inflammation, especially a pathologic role for IL-1β in cardiovascular disease. Inflammasomes play protective roles in host defense, however the activation can also contribute to ischemia-reperfusion injury (I/RI) and autoinflammation. APC has been shown to restrict inflammasome activation after myocardial I/RI in a mouse model, thus demonstrating that APC is anti-inflammatory in the Inflammasome process.

In some embodiments, a cultured human THP-1 cell line is employed in the screening methods of the invention. THP-1 cells are a human monocytic cell line, capable of being differentiated into macrophages. As demonstrated herein, using select agonists in THP-1 cells is an effective in vitro means to induce the Inflammasome and/or caspase I activity and ultimately, inflammation. These screening methods of the invention entail inducing inflammation that is Inflammasome- and/or caspase I-dependent in THP-1 cells via an inflammation inducing agent (e.g., LPS as exemplified herein), and assessing potential anti-inflammatory activities of candidate agents against that of an exemplified anti-inflammatory peptide (e.g., P3R or TR47). Novel anti-inflammatory agents can be identified if they possess similar or stronger activities in suppressing inflammation evidenced by an inflammation related activity or a phenotype manifesting Inflammasome activation and/or caspase 1 activity, e.g., caspase 1 activity or secretion of IL-1β.

The candidate agents that can be used in the screening methods of the invention can be of any chemical nature. These include, e.g., peptides, include polypeptides, beta-turn mimetics, polysaccharides, phospholipids, hormones, prostaglandins, steroids, purines, pyrimidines, oligomeric N-substituted glycines, oligocarbamates, saccharides, fatty acids, as well as derivatives, structural analogs or combinations thereof. In some embodiments, the candidate agents to be used in the screening methods of the invention are small molecule organic compounds. In some of these embodiments, combinatorial libraries of small molecule candidate agents can be employed to screen for therapeutic agents. Such compound libraries are well known in the art, e.g., as described in Schultz et al., Bioorg. Med. Chem. Lett. 8:2409-2414, 1988; Weller et al., Mol Divers. 3:61-70, 1997; Fernandes et al., Curr. Opin. Chem. Biol. 2:597-603, 1998; and Sittampalam et al., Curr. Opin. Chem. Biol. 1:384-91, 1997. Candidate agents include unringed and unbranched small organic molecules, as well as other organic compounds such as aromatic compounds, heterocyclic compounds, and benzodiazepines.

In some embodiments, the candidate agents to be screened in the methods of the invention are PAR3- or PAR1- derived peptides or polypeptides, variants, analogs, peptidomimetics or other related or derivative compounds described herein. In some of these embodiments, the candidate agents to be screened with methods of the invention can be variants, derivative or mimetic compounds of peptides or polypeptides mimicking the N-terminal sequence of Met¹-Arg⁴¹ deleted extracellular domain of human PAR3 (SEQ ID NO:3) or Met¹-Arg⁴⁶ deleted extracellular domain of human PAR1 (SEQ ID NO:6) described herein. For examples, the candidate agents can be polypeptides derived from SEQ ID NO:3 or SEQ ID NO:6 with various C-terminal or internal deletions. In some embodiments, the P3R peptide (SEQ ID NO:4) or the TR47 peptide (SEQ ID NO:7) can be used as a scaffold to generate a library of variant or analog peptides and peptidomimetics. The library of candidate agents based on a reference peptide or polypeptide (e.g., P3R peptide) can be readily produced using routinely practiced methods as described herein. Relative to the reference peptide (e.g., P3R or TR47) or polypeptide (e.g., SEQ ID NO: 3 or 6), the candidate agents can also be a library of variant peptides or polypeptides that contain one or more amino acid substitutions.

Methods for preparing libraries containing diverse populations of peptides, peptoids and peptidomimetics are well known in the art and various libraries are commercially available. See, e.g., Ecker and Crooke, Biotechnology 13:351-360, 1995; and Blondelle et al., Trends Anal. Chem. 14:83-92, 1995; and the references cited therein. See, also, Goodman and Ro, Peptidomimetics for Drug Design, in "Burger's Medicinal Chemistry and Drug Discovery" Vol. 1 (ed. M. E. Wolff; John Wiley & Sons 1995), pages 803-861; and Gordon et al., J. Med. Chem. 37:1385-1401 (1994). One skilled in the art understands that a peptide can be produced in vitro directly or can be expressed from a nucleic acid, which can be produced in vitro. A library of peptide molecules also can be produced, for example, by constructing a cDNA expression library from mRNA collected from a tissue of interest. Methods for producing such libraries are well known in the art (see, for example, Sambrook et. al., Molecular Cloning: A laboratory manual (Cold Spring Harbor Laboratory Press 1989).

Methods of designing peptide derivatives and mimetics and screening of functional peptide mimetics are well known to those skilled in the art. One basic method of designing a molecule which mimics a known protein or peptide is first to identify the active region(s) of the known protein (for example, in the case of an antibody-antigen interaction, one identifies which region(s) of the antibody that permit binding to the antigen), and then searches for a mimetic which emulates the active region. Although the active region of a known polypeptide is relatively small, it is anticipated that a mimetic will be smaller (e.g., in molecular weight) than the protein, and correspondingly easier and cheaper to synthesize and/or have benefits regarding stability or other advantageous pharmacokinetic aspects. Such a mimetic could be used as a convenient substitute for the reference polypeptide (e.g., SEQ ID NO:3) or peptide (e.g., P3R), as an agent for interacting with the target molecule (e.g., PAR3). For example, Reineke et al. (Nat. Biotech. 17; 271-275, 1999) designed a mimic molecule which mimics a binding site of the interleukin-10 protein using a large library of short synthetic peptides, each of which corresponded to a short section of interleukin 10. The binding of each of these peptides to the target (in this case an antibody against interleukin-10) was then tested individually by an assay technique, to identify potentially relevant peptides. Phage display libraries of peptides and alanine scanning methods can be used.

Other methods for designing peptide mimetics to a particular peptide or protein include those described in European Patent EP1206494, the SuperMimic program by Goede et. al., BMC Bioinformatics, 7:11, 2006; and MIMETIC program by Campbell et al., Microbiol. and Immunol. 46:211-215, 2002. The SuperMimic program is designed to identify compounds that mimic parts of a protein, or positions in proteins that are suitable for inserting mimetics. The application provides libraries that contain peptidomimetic building blocks on the one hand and protein structures on the other. The search for promising peptidomimetic linkers for a given peptide is based on the superposition of the peptide with several conformers of the mimetic. New synthetic elements or proteins can be imported and used for searching. The MIMETIC computer program, which generates a series of peptides for interaction with a target peptide sequence, is taught by W. Campbell et. al., 2002. In depth discussion of the topic is reviewed in "Peptide Mimetic Design with the Aid of Computational Chemistry" by James R. Damewood Jr. in Reviews in Computational Chemistry, January 2007, Volume 9, Editor(s): Kenny B. Lipkowitz, Donald B. Boyd (John Wiley &Sons, Inc.); and in Tselios, et. al., Amino Acids, 14: 333-341, 1998.

Once a library of candidate agents is prepared, they can be readily screened for optimized or improved anti-inflammatory activities relative to the activities of the reference polypeptide. The candidate agents can be screened for improvement in any of the anti-inflammatory activities of the reference polypeptide disclosed herein, e.g., inhibition or suppression of caspase 1 enzymatic activity or secretion of IL-1β (see Examples below). Variants or analog compounds based on a reference PAR3- or PAR1-derived anti-inflammatory peptide (e.g., P3R or TR47) which are optimized with such a screening method can be employed in the various therapeutic applications described herein.

### VII. Therapeutic compositions and dosages

The invention provides therapeutic or pharmaceutical compositions for use in the practice of the therapeutic or prophylactic methods described herein. The PAR3- and/or PAR1- derived anti-inflammatory peptides and related compounds described herein (e.g., variants, derivatives and mimetics) that possess anti-inflammatory activities, and the other therapeutic agents disclosed herein can be administered directly to subjects in need of treatment. However, these therapeutic compounds are preferably administered to the subjects in pharmaceutical compositions which comprise the peptides, variants or mimetics, and/or other active agents along with a pharmaceutically acceptable carrier, diluent or excipient in unit dosage form. Accordingly, the invention provides pharmaceutical or therapeutic compositions comprising one or more of the anti-inflammatory peptides or derivative compounds disclosed herein. The invention also provides a use of these peptides or related compounds in the preparation of pharmaceutical compositions or medicaments for treating or preventing the above described diseases or medical disorders that are mediated by or associated with undesired inflammation.

Some compositions of the invention contain one PAR3-derived anti-inflammatory peptide or a related compound as described herein (e.g., a variant, derivative and mimetic). Some of these compositions are pharmaceutical compositions that contain a therapeutically effective amount or dosage of a PAR3-derived anti-inflammatory peptide described herein (e.g., P3R) or a derivative compound, and a pharmaceutically acceptable carrier. Some compositions of the invention contain one PAR1-derived anti-inflammatory peptide or a related compound as described herein. Some of these compositions are pharmaceutical compositions that contain a therapeutically effective amount or dosage of a PAR1-derived anti-inflammatory peptide described herein (e.g., TR47) or a derivative compound, and a pharmaceutically acceptable carrier.

Still come other compositions of the invention contain both a PAR3-derived anti-inflammatory peptide or related compound and a PAR1-derived anti-inflammatory peptide or related compound as described herein. Some of these compositions are pharmaceutical composition contains a therapeutically effective amount or dosage of both a PAR3-derived anti-inflammatory peptide and a PAR1-derived anti-inflammatory peptide described herein, or related or derivative compounds described herein, plus a pharmaceutically acceptable carrier. As described above, the PAR3-derived anti-inflammatory peptide and the PAR1-derived anti-inflammatory peptide can be provided as separate peptides, in monomeric or multimeric forms, in the composition. Alternatively, the peptides can be conjugated to each other as a heterodimer or hetero-multimer in the composition as described above. In various embodiments, the monomeric, hetero- or homo-dimeric, and hetero- or homo-multimeric peptides in the composition can be further conjugated, by covalent or non-covalent linkage, to one or more carrier moieties described herein. In some of the compositions, the PAR3-derived peptide or related compound and the PAR1-derived peptide or related compound can be conjugated to the carrier moiety at varying ratios as described herein.

Pharmaceutically acceptable carriers are agents which are not biologically or otherwise undesirable. These agents can be administered to a subject along with the PAR3- and/or PAR1-derived peptides without causing any undesirable biological effects or interacting in a deleterious manner with any of the components of the pharmaceutical composition. The compositions can additionally contain other therapeutic agents that are suitable for treating or preventing undesired inflammation or inflammatory disorders. Pharmaceutically carriers enhance or stabilize the composition or facilitate preparation of the composition. Pharmaceutically acceptable carriers include solvents, dispersion media, coatings, antibacterial and antifungal agents, isotonic and absorption delaying agents, and the like that are physiologically compatible. The pharmaceutically acceptable carrier employed should be suitable for various routes of administration described herein. Additional guidance for selecting appropriate pharmaceutically acceptable carriers is provided in the art, e.g., Remington: The Science and Practice of Pharmacy, Mack Publishing Co., 20th ed., 2000. In addition to the anti-inflammatory peptides and the pharmaceutically acceptable carriers, the pharmaceutical compositions of the invention can further contain other active or inactive agents for suppressing information and known drugs for treating the specific inflammatory disorder. For example, the pharmaceutical compositions can include known anti-inflammatory agents and analgesics. Examples of such agents include glucocorticoids, non-steroidal anti-inflammatory drug (NSAIDs), cetaminophen, opiates, diproqualone, and lidocaine topical.

A pharmaceutical composition containing the PAR3- and/or PAR1-derived anti-inflammatory peptides or derivative compounds described herein and/or other therapeutic agents can be administered by a variety of methods known in the art, e.g., oral administration. The routes and/or modes of administration vary depending upon the desired results. Depending on the route of administration, the active therapeutic agent may be coated in a material to protect the compound from the action of acids and other natural conditions that may inactivate the agent. Conventional pharmaceutical practice may be employed to provide suitable formulations to administer such compositions to subjects. Any appropriate route of administration may be employed. These include, but are not limited to, oral, intravenous, parenteral, transcutaneous, subcutaneous, intraperitoneal, intramuscular, intracranial, intraorbital, intraventricular, intrapulmonary, intracapsular, and intraspinal administration. Depending on the specific conditions of the subject to be treated, either systemic or localized delivery of the therapeutic agents may be used in the treatment.

When administered to a subject in vivo, the pharmaceutical compositions typically contain a therapeutically effective amount or dosage of the active anti-inflammatory compounds. A therapeutically effective amount is the total amount of the anti-inflammatory compound that achieves the desired anti-inflammatory effect. Effective dosages or doses vary depending upon many different factors, including means of administration, target site, physiological state of the patient, whether the patient is human or an animal, other medications administered, and whether treatment is prophylactic or therapeutic. Usually, the patient is a human but nonhuman mammals can also be treated. Treatment dosages need to be titrated to optimize safety and efficacy. As a general guidance, the dosage ranges from about 0.0001 to 100 mg/kg, and more usually 0.01 to 5 mg/kg, of the host body weight. For example dosages can be 1 mg/kg body weight or 10 mg/kg body weight or within the range of 1-10 mg/kg.

In some embodiments, each peptide or derivative compound in the composition is provided at a concentration of at least 10 nM, 25 nM, 50 nM, 100 nM, 250 nM, 500 nM, 1 µM, 2.5 µM, 5 µM, 10 µM, 25 µM or higher. Depending on the specific circumstances, each administration to the subject may include a dosage of about 0.5 ml, 1 ml, 2.5 ml, 5 ml, 10 ml, 25 ml, 50 ml, 100 ml or more of the composition. In some embodiments, dosage for each administered peptide or derivative compound can range from about 10 ng to 1 g, 100 ng to 100 mg, 1 µg to 10 mg, or 30-300 µg per patient. In some embodiments, dosage for each administered anti-inflammatory peptide or derivative is adjusted to achieve a plasma concentration of 1-1000 µg/ml, and in some embodiments 25-300 µg/ml. In some embodiments, dosage for each administered anti-inflammatory peptide or derivative can be adjusted to achieve a plasma concentration of about 0.01 µg/ml to about 1.6 µg/ml, preferably from about 0.01 µg/ml to about 0.5 µg/ml. It is also within the skill of the art to start doses at levels lower than required to achieve the desired therapeutic effect and to gradually increase the dosage until the desired effect is achieved. It is likewise within the skill of the art to determine optimal concentrations of variants to achieve the desired effects in the in vitro and ex vivo preparations of the invention. Depending on initial assay results, optimal concentrations can be in the range of, e.g., about 1-1,000 nM or about 1-200 µM depending on the general nature of the compound.

Exemplary treatment regimens entail administration once per day, once every other day, once every week, once every two weeks, once a month or once every 3 to 6 months. In some methods, two or more anti-inflammatory peptides (e.g., P3R and TR47) or their respective derivatives are administered simultaneously, in which case the dosage of each therapeutic peptide administered falls within the ranges indicated. The pharmaceutical composition is usually administered on multiple occasions. As noted above, intervals between single dosages can be weekly, monthly or even yearly. Intervals can also be irregular as indicated by measuring blood levels of the anti-inflammatory peptides or peptide-containing derivative compound (e.g., a complex containing a peptide and a carrier moiety) in the patient. Alternatively, the PAR3- and/or PAR1-derived peptides can be administered as a sustained release formulation, in which case less frequent administration is required. Dosage and administration frequency can also vary depending on the half-life of the administered compound in the patient. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patent can be administered a prophylactic regime.

Some methods of the invention involve the administration of a combination of a PAR3-derived anti-inflammatory peptide and a PAR1-derived anti-inflammatory peptide, or their respective derivative compounds described herein. As exemplified herein with peptides P3R and TR47, a very low dose of one of the two peptides is sufficient to achieve a synergistic effect. Thus, in some embodiments, each of the two peptides is provided in the pharmaceutical composition at a concentration of at most about 500 nM, 250 nM, 50 nM, 25 nM, 10 nM, 5 nM, 2.5 nM, 1 nM or lower. In some embodiments, administration of the pharmaceutical composition provides each of the two peptides with a daily dosage of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of the host body weight. Depending on the average body weight of the subject (human or non-human animal, adults or youngsters), an appropriate amount (volume) of the composition is administered to the subject in order to achieve the desired total daily dosages described herein. Thus, for example, an average body weight of 75 kg may be used to calculate the total amount of the peptides for each daily administration to adult human subjects or animals with similar body weight. Similarly, an average body weight of 60 kg, 50 kg, 40 kg, 30 kg, 20 kg, 15 kg, 10 kg, 7.5 kg, 5 kg or lower may be used calculate the total amount of the peptides for each daily administration to non-adults of different age groups (e.g., age groups of 15-18, 11-14, 7-10, 4-6, 2-3, 1-2 or younger) or animals with similar body weights.

In some other embodiments, the pharmaceutical composition can contain one peptide (e.g., a PAR3-derived peptide) or derivative compound at a dosage that is much higher than that of the other peptide (e.g., a PAR1-derived peptide) or derivative compound. For example, the composition to be administered to the subject can contain one peptide (e.g., P3R or a P3R variant or derivative) at a concentration that is at least about 10 nM, 25 nM, 50 nM, 100 nM, 250 nM, 500 nM, 1 µM, 2.5 µM, 5 µM, 10 µM, 25 µM or higher. The other peptide (e.g., TR47 or a TR47 variant or derivative) in such a composition can be at a concentration of at most about 500 nM, 250 nM, 50 nM, 25 nM, 10 nM, 5 nM, 2.5 nM, 1 nM or lower. In some of these embodiments, dosage of one of the two peptides to be administered to the subject can be a daily amount of at least about 0.01 mg/kg, 0.025 mg/kg, 0.05 mg/kg, 0.1 mg/kg, 0.25 mg/ kg. 0.5 mg/kg, 1 mg/kg, 2.5 mg/kg, 5 mg/kg, 10 mg/kg, 25 mg/kg or higher, of the host body weight. Dosage of the other peptide in the pharmaceutical composition for these embodiments can be a daily amount of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of the host body weight. Again, the total amount of the peptides in the compositions for use in these embodiments can be determined in accordance with the average body weight of the subject. For example, an average body weight of 75 kg may be used to calculate the total amount of the peptides for each daily administration to adult human subjects or animals with similar body weight. Similarly, an average body weight of 60 kg, 50 kg, 40 kg, 30 kg, 20 kg, 15 kg, 10 kg, 7.5 kg, 5 kg or lower may be used calculate the total amount of the peptides for each daily administration to non-adults of different age groups (e.g., age groups of 15-18, 11-14, 7-10, 4-6, 2-3, 1-2 or younger) or animals with similar body weights.

Pharmaceutical compositions of the invention can be prepared in accordance with methods well known and routinely practiced in the art. See, e.g., See, e.g., Goodman & Gilman's The Pharmacological Bases of Therapeutics, Hardman et al., eds., McGraw-Hill Professional (10th ed., 2001); Remington: The Science and Practice of Pharmacy, Gennaro, ed., Lippincott Williams & Wilkins (20th ed., 2003); Pharmaceutical Dosage Forms and Drug Delivery Systems, Ansel et al. (eds.), Lippincott Williams & Wilkins (7th ed., 1999); and Sustained and Controlled Release Drug Delivery Systems, J.R. Robinson, ed., Marcel Dekker, Inc., New York, 1978. Pharmaceutical compositions are preferably manufactured under GMP conditions.

### EXAMPLES

The following examples are provided to further illustrate the invention but not to limit its scope. Other variants of the invention will be readily apparent to one of ordinary skill in the art and are encompassed by the appended claims.

### Example 1 Synergistic anti-inflammatory activities of PAR1- and PAR3-derived peptides

We first observed that APC treatment reduced caspase 1 activity in human PMA-differentiated, THP1-null cells that were treated with LPS and ATP (Figure 1A). APC exhibits biased signaling by cleaving PAR1 at the non-canonical residue Arg46, and the PAR1-derived 20 amino acid peptide, TR47, mimicking the novel N-terminus derived from Arg46 cleavage induces protective signaling in endothelial cells in vitro and reduces vascular leakage *in vivo.* TR47 at 50 µM was initially tested based off previously *in vivo* published data, and we found that 50 µM TR47 as well as lower concentrations similarly reduced caspase 1 activity, whereas neither a scrambled TR47 nor the peptides, SFLLRN or TFLLRN which resemble the novel N-terminal amino acid sequence generated by thrombin's PAR1 cleavage at Arg41 had any effect on caspase 1 activity (Figure 1A). Protective signaling by APC can follow its cleavage of PAR3 at Arg41 on endothelial cells, and the PAR3-derived peptide, P3R, results in protective effects *in vivo* and reduced *in vitro* neutrophil extracellular trap formation. Similar to APC and TR47, the P3R peptide reduced caspase 1 activity. A control peptide, P3K, the PAR3 peptide representing thrombin cleavage at Lys38, did not significantly change caspase 1 activity in THP1 cells (Figure 1B).

We hypothesized that although low concentrations of either TR47 or P3R is insufficient to exert anti-inflammatory activity by reducing caspase 1 activity, the combination of these two GPCR agonists could cooperatively reduce caspase 1 activity. When TR47 at <10 nM in the presence of 500 nM P3R was incubated with THP1 cells prior to induction of caspase 1 activity, a reduction in caspase 1 activity was observed, in contrast to TR47's effects in the absence of P3R after normalizing to caspase 1 inhibited conditions (Figure 1C & 1D). Furthermore, when P3R at concentrations ≤500 nM was incubated with 1 nM TR47, a significant reduction in normalized caspase 1 activity was observed, in contrast to P3R in the absence of TR47 (Figure 1E). Whether the intracellular signaling initiated by APC, TR47, P3R or peptide combination is similar or conserved across various human cell types remains to be resolved. The anti-inflammatory synergistic effects of TR47 and P3R suggests that PAR1 and PAR3 might exist as a heterodimeric GPCR complex within an interactome that promotes anti-inflammatory intracellular signaling. It is tempting to imagine that linking these two GPCR agonist peptides might be therapeutically promising in select inflammatory diseases.

Additional studies were performed to further substantiate the observed synergistic activities. In these studies, additional variations of PAR peptides that were made and shown to retain their anti-inflammatory activities (Figures 4, 5, and 7A) were tested for retention of cooperativity for anti-inflammatory activity (Figure 7) as shown for the PAR1-derived TR47 and the PAR3-derived P3R combinations (Figure 1D-E). For one example of such studies, consider the PAR3 peptide P3(51-65) (aka peptide "P3R51-65" herein) which alone is anti-inflammatory at relatively high concentrations, e.g., at 50 µM. When it was tested at concentrations of 0 to 16 nM in the presence or absence of 1 nM peptide P1(47-66) (aka peptide "TR47" herein), only in the presence of 1 nM P1(47-66) was a significant reduction in caspase-1 activity seen (Figure 7D), as observed for peptide P3(42-65) (aka peptide "P3R" herein) (Figure 1E). These findings (Figure 7B-D) indicate that variations of the PAR3 peptide ligand from the N terminus or changing the sequence or length can result in a cooperative, synergistic anti-inflammatory response.

Data in Figure 7B-D show that combinations of different PAR1-derived and PAR3-derived peptides provide enhanced potency for anti-inflammatory activity compared to the anti-inflammatory activity of any single peptide alone that was used for a given studied combination. For biologic potency, this emphasizes the value of combinations of various different PAR1-derived and PAR3-derived peptides versus each peptide alone. These data indicate that variations of PAR peptide's sequence or length can be made and still result in cooperative, synergistic anti-inflammatory responses (Figure 1D-E, and Figure 7B-D).

### Example 2 Mechanism of anti-inflammatory activities of PAR1- and PAR3-derived peptides

NLRP3 is a canonical sensor protein central to inflammasome formation, and, notably, mutations in NLRP3 result in chronic autoinflammatory disease in humans.²³ NLRP3-deficient (defNLRP3) THP1 cells or inhibition of NLRP3 using the NLRP3 inhibitor, MCC950, for THP1-null cells resulted in a significant reduction in caspase 1 activity across all treatments tested (Figure 2A & 2B). These data show that generation of the caspase 1 activity which is inhibited by APC, TR47, and P3R requires NLRP3.

For noncanonical, biased signaling induced by APC, EPCR is an important APC-binding cell receptor necessary for non-canonical cleavages of PAR1 and PAR3. Blocking EPCR with the anti-EPCR monoclonal antibody RCR-252 prior to APC or TR47 treatment resulted significant loss in their inhibition of caspase 1 activity, whereas the P3R study, though trending, did not reach significance (Figure 2C). To test whether PAR3 contributed to the anti-inflammatory effects of APC, TR47 and P3R, a blocking antibody was used to inhibit PAR3 prior to treatment.^{16,26} Blocking PAR3 very significantly reduced the anti-inflammatory activity of APC and P3R and less significantly reduced the activity of TR47 (Figure 2D). APC-initiated signaling via PAR1 is widely recognized as critical for beneficial effects in many cell types. Inhibition of PAR1 using either blocking monoclonal antibodies, WEDE15 and ATAP2, or the PAR1 small molecule antagonist SCH79797 (SCH),¹⁷ significantly inhibited the reduction of caspase 1 activity by APC (Figure 2E). TR47's action was inhibited by SCH79797 and WEDE15, but not by ATAP2; however, the WEDE15 epitope comprises in part the sequence of TR47. None of the PARI-targeted inhibitors statistically significantly reduced the anti-inflammatory activity of the PAR3 peptide agonist, P3R (Figure 2E & 2F).

IL-1β is a key cytokine generated by NLRP3 inflammasomes. The generation and release of IL-1β by THP1 cells subjected to protocols as described above for caspase 1 activity was significantly suppressed by APC, TR47, P3R and the combination of TR47 and P3R (Figure 3). This confirms that these agents are anti-inflammatory based on the reduction of two widely recognized biomarkers of inflammation.

### Example 3 Structural requirement for anti-inflammatory activities of PAR1- and PAR3-derived peptides

To define the relationship between the sequence and activity of the peptides, we next sought to test peptides of various lengths, beginning from the N-terminus or C-terminus. First, we tested P3R peptide variants, beginning with the previously described 13-mer P3Rmed (P3Rm).¹⁶ At 50 µM, similar to the 24-mer P3R (residues 42-65; 24-mer), a significant reduction in caspase 1 activity was observed, and this effect was lost at 2 µM (Figure 4A-C). Subsequently, shortening the peptide from the N terminus, both P3R 51-65 or 51-59 exhibited similar reductions in caspase 1 activity at 50 µM that were lost at 2 µM (Figure 4). Peptides that were further shortened by 3 or 8 amino acids (P3R 54-59 or 59-65, respectively) only partially reduced caspase 1 activity at 50 µM (Figure 4A), suggesting that the Phe, Pro, Phe sequence might be critical for anti-inflammatory activity (Figure 4C) and the cryptic N-terminal for PAR3 after APC-mediated cleavage might not be the critical signaling portion, potential challenging the paradigm of PAR activation and signaling, at least for PAR3.

Next, we sought to define the sequence critical to mediate TR47-mediated reduction in caspase 1 activity. Our initial experiments demonstrate that the previously published 9-mer (NPNDKYEPF; SEQ ID NO:36) of TR47, at 50 µM reduced caspase 1 activity similar to the 20-mer TR47 (Figure 5A). In contrast, when the N-terminus of TR47 was acylated, no reduction in caspase 1 activity was observed at equimolar concentrations, suggesting a free amine N-terminus might be critical for TR47-mediated anti-inflammatory activity. Next, we tested additional C-terminus shortened TR47 peptides, including the previously published 8-mer TR47 (NPNDKYEP; SEQ ID NO:35), which similar to the 16-mer TR47 significantly reduced caspase 1 activity, whereas the 6-mer TR47 lost its inhibitory activity suggesting peptides longer than 6 amino acids are necessary for anti-inflammatory activity for PARI (Figure 5B). Next, we sought to test the paradigm that the N-terminus Asn (N) is required for TR47's anti-inflammatory activity. The loss of 1 amino acid from the N-terminus (TR47 N-1) still reduced caspase 1 activity, whereas removal of 2 amino acids (TR47 N-2) resulted in a loss in inhibition of caspase 1 activity. Curiously, a reduction in caspase 1 activity was observed with 5 amino acids removed from the N-terminus (TR47 N-5), suggesting that the cryptic N-terminal of PAR1 after APC-mediated cleavage is not entirely responsible for the beneficial signaling effects (Figure 5B). Next, point mutations of the N-terminus residue 47 demonstrated that the charge of the N-terminus had variable effects for TR47-mediated reductions in caspase 1 activity. Mutation of N → Q (Asn to Gin, one carbon-carbon bond longer on the side chain) or N → A in TR47 gave peptides that similarly reduced caspase 1 activity. However, the mutation of residue 47's N → D (Asn to Asp) resulted in a loss in reduction in caspase 1 activity (Figure 5B). Finally, a truncated TR-peptide, removing both one amino acid from the N-terminus and part of the C-terminus, TR48-54, did not reduce caspase 1 activity, suggesting that both a minimum length requirement for the peptide, and potentially different recognition internal sequences are needed for the full anti-inflammatory effects of TR47 observed (Figure 5B-C).

Synergistic anti-inflammatory activities of the variant peptides were also examined. Some exemplary results are shown in Figure 6. These include synergy observed with a combination of PAR1 derived Peptide TR47 and PAR3 derived Peptide P3R variant P3Rm (Figure 6A) and a combination of PAR1 derived Peptide TR47 variant TR47 (N-1) and PAR3 derived Peptide P3R (Figure 6B).

### Example 4 Anti-inflammatory activity of a PAR1 peptide/PAR3 peptide conjugate

This Example describes studies showing that covalent linkage of a PAR1 peptide and a PAR3 peptide improves anti-inflammatory activity.

PAR1 9-mer peptide, P1(47-55) (residues 47-55; SEQ ID NO:36), and the PAR3 peptide, P3(51-65) (SEQ ID NO:9), each of which alone is anti-inflammatory at relatively high concentrations were used. To demonstrate the anti-inflammatory efficacy of covalently linked PAR1-derived and PAR3-derived peptides, a PAR1/PAR3 fusion peptide ("G10") was generated by covalently linkage with a linker comprised of 10 Glycine residues, NPNDKYEPFGGGGGGGGGGFPFSALEGWTGATIT (SEQ ID NO:61). The fusion peptide together with its two component peptides were then tested for anti-inflammatory activities.

At concentrations tested (0-500 nM), a significant reduction in caspase-1 activity in the presence of the "G10" peptide was observed at 2 nM - 20 nM whereas neither the isolated PAR1-derived peptide nor the isolated PAR3-derived peptide had an any anti-inflammatory effect at 200 nM (Figure 8). As expected, at relatively higher concentrations, the P1(47-55) peptide alone was anti-inflammatory at 500 nM (Figure 8).

The results further underscore the synergistic activity of the PAR1 and PAR3 agonist peptides. While each of the two peptides independently is anti-inflammatory, the combination is more potent than either peptide alone.

### Example 5 PAR1/PAR3 fusion peptide promotes endothelial barrier stability

This Example describes studies showing that PARI-derived, PAR3-derived and covalently linked PAR1/PAR3 fusion ("G10") peptides inhibit thrombin-induced disruption of endothelial barrier.

Transendothelial Electric Resistance (TER)-based assays were used to monitor the disruption of endothelial barrier integrity caused by thrombin. The anti-inflammatory, vasculoprotective actions of different PAR peptides for protecting against thrombin-induced vascular leakage on cultured endothelial cells (EA.hy926) were followed in real time using an iCELLigence machine (ACEA Biosciences Inc, San Diego, CA). EA.hy926 cells at a density of 10,000/well were pipetted into the wells of gold-plated microelectrode sensor (L8, Acea Biosciences). Subsequently, the increase of electrical resistance was monitored as cells grew and spread out on the microelectrodes to obtain confluence (reaching a plateau) over the TER plates connected to the recording instrument that detected in real time the changes in TER for each well. The effects of different PAR-derived peptides at the indicated concentrations were tested for dose-response inhibition of thrombin's effects when each of the peptide dilutions was added 30 min prior to the addition of thrombin (0.25 nM final in all wells). The percentage of inhibition was proportional to the increase in TER signal over time. Cell index values, reflecting TER signals, were normalized prior to addition of thrombin following manufacturer's directions.

Results from the study are shown in Figure 9. The results indicate that the heterobivalent PAR1:PAR3 agonist peptide, G10, showed remarkable potency for its ability to stabilize endothelial cell barrier integrity, which was similar to its potent anti-inflammatory activity determined by ability to reduce caspase-1 activity (Figure 8) with half-maximum observed activity at approximately 6 nM G10 peptide (SEQ ID NO:61). In Figure 9, the G10 peptide was ~ 8-fold more potent than the long PAR1 peptide TR47 (e.g., compare effect of 6 nM G10 (Figure 9E) to effect of 50 nM PAR1 peptide P1(47-56) alone (Figure 9A) and the G10 peptide (Figure 9E) was > 100-fold more effective than PAR3 peptide P3(42-54) alone (Figure 9D) for the protection of endothelial barrier integrity. Another PAR3 peptide, P3(42-65) that includes the 47-55 sequence had no significant effect when tested at 5 to 50 nM (Figure 9B) compared to the very significant effects of 3 to 50 nM peptide G10 (Figure 9E).

### Example 6 Some exemplified methods

Caspase 1 assay. THP1-null (wildtype cells) or NLRP3-deficient THP1-(defNLRP3) cells were plated at a final concentration of 1×10⁶/mL in 96 well plates and incubated with PMA (0.5 µM) for 3 hours 37°C in supplemented RPMI. Media was subsequently changed every 24 hours for 3 days. Various subsets of wells were selected and cells were treated for 60 minutes at 37°C in serum-free RPMI with various concentrations of APC, TR47 or P3R. Following DPBS wash, cells were incubated with LPS (lipopolysaccharide) (1 µg/mL) for 3 hours at 37°C in serum-free RPMI to prime the inflammasome. After a DPBS wash, to fully activate caspase 1 activity, cells were incubated with ATP (5 mM), in the presence or absence of 10 µM YVAD (caspase 1 inhibitor), and 2.5 µM ZVAD (pan-caspase inhibitor) for 45 minutes at 37°C, prior to performing the caspase 1 activity assay following the manufacturer's (Promega) directions; caspase 1 activity was monitored as luminescence changes due to substrate hydrolysis and reported as relative luminescent units (RLU).¹⁵ In select experiments, caspase 1 activity of experimental conditions were normalized to YVAD (caspase 1 inhibitor) conditions and expressed as normalized caspase 1 activity.

IL-1β ELISA. THP1 cells were plated at a final concentration of 1 × 10⁶/mL in 96 well plates and incubated with PMA (0.5 µM) for 3 hours 37°C in supplemented RPMI. Media was subsequently changed every 24 hours for 3 days. Selected wells were treated with APC (4 µg/mL), TR47 (50 µM), P3R (50 µM) or the combination of TR47/P3R (8 nM & 500 nM, respectively) for 1 hour. Cells were then washed with DPBS, then incubated with LPS (1 µg/mL) for 3 hours at 37°C in serum-free RPMI. Cells were washed with DPBS and incubated with ATP (5 mM) for 45 minutes. Cell supernatants were carefully collected and frozen until assayed using the IL-1β Quantikine ELISA following the manufacturer's (R&D Systems) directions, measuring absorbance. The concentration of IL-1β was calculated following measurement of the standard curve according to the manufacturer's directions.

Although the foregoing invention has been described in some detail by way of illustration and example for purposes of clarity of understanding, it will be readily apparent to one of ordinary skill in the art in light of the teachings of this invention that certain changes and modifications may be made thereto without departing from the spirit or scope of the appended claims.

All publications, databases, GenBank sequences, patents, and patent applications cited in this specification are herein incorporated by reference as if each was specifically and individually indicated to be incorporated by reference.

Some exemplified polypeptide sequences
SEQ ID NO:1. Human PAR3 
MKALIFAAAG LLLLLPTFCQ SGMENDTNNL AKPTLPIKTF RGAPPNSFEE
FPFSALEGWT GATITVKIKC PEESASHLHV KNATMGYLTS SLSTKLIPAI
YLLVFVVGVP ANAVTLWMLF FRTRSICTTV FYTNLAIADF LFCVTLPFKI
AYHLNGNNWV FGEVLCRATT VIFYGNMYCS ILLLACISIN RYLAIVHPFT
YRGLPKHTYA LVTCGLVWAT VFLYMLPFFI LKQEYYLVQP DITTCHDVHN
TCESSSPFQL YYFISLAFFG FLIPFVLIIY CYAAIIRTLN AYDHRWLWYV
KASLLILVIF TICFAPSNII LIIHHANYYY NNTDGLYFIY LIALCLGSLN
SCLDPFLYFL MSKTRNHSTA YLTK
SEQ ID NO:2. Human PAR3 Met¹-Arg⁴¹ deleted fragment 
GAPPNSFEE
FPFSALEGWT GATITVKIKC PEESASHLHV KNATMGYLTS SLSTKLIPAI
YLLVFVVGVP ANAVTLWMLF FRTRSICTTV FYTNLAIADF LFCVTLPFKI
AYHLNGNNWV FGEVLCRATT VIFYGNMYCS ILLLACISIN RYLAIVHPFT
YRGLPKHTYA LVTCGLVWAT VFLYMLPFFI LKQEYYLVQP DITTCHDVHN
TCESSSPFQL YYFISLAFFG FLIPFVLIIY CYAAIIRTLN AYDHRWLWYV
KASLLILVIF TICFAPSNII LIIHHANYYY NNTDGLYFIY LIALCLGSLN
SCLDPFLYFL MSKTRNHSTA YLTK
SEQ ID NO:3 Human PAR3 Met¹-Arg⁴¹ deleted extracellular domain GAPPNSFEE FPFSALEGWT GATITVKIKC PEESASHLHV KNATMGYLTS SLST
SEQ ID NO:4. PAR3 derived peptide P3R (first 24 residues of SEQ ID NO:3) GAPPNSFEE FPFSALEGWT GATIT
SEQ ID NO:5. Human PAR1 Met¹-Arg⁴⁶ deleted fragment 
47 NPND KYEPFWEDEE
61 KNESGLTEYR LVSINKSSPL QKQLPAFISE DASGYLTSSW LTLFVPSVYT
111 GVFVVSLPLN IMAIVVFILK MKVKKPAVVY MLHLATADVL
151 FVSVLPFKIS YYFSGSDWQF GSELCRFVTA AFYCNMYASI
191 LLMTVISIDR FLAVVYPMQS LSWRTLGRAS FTCLAIWALA
 
231 IAGVVPLLLK EQTIQVPGLN ITTCHDVLNE TLLEGYYAYY
271 FSAFSAVFFF VPLIISTVCY VSIIRCLSSS AVANRSKKSR ALFLSAAVFC
321 IFIICFGPTN VLLIAHYSFL SHTSTTEAAY FAYLLCVCVS SISCCIDPLI
371 YYYASSECQR YVYSILCCKE SSDPSSYNSS GQLMASKMDT
411 CSSNLNNSIY KKLLT
SEQ ID NO:6. Human PAR1 Asn⁴⁷-Trp¹⁰⁰ (Met¹-Arg⁴⁶ deleted extracellular domain) NPND KYEPFWEDEE KNESGLTEYR LVSINKSSPL QKQLPAFISE DASGYLTSSW
SEQ ID NO:7. Human PAR1 derived peptide TR47 (first 20 residues of SEQ ID NO:6) NPND KYEPFWEDEE KNESGL

### CLAUSES

**1.** A method for suppressing an undesired inflammation and/or treating an inflammatory condition in a subject, comprising administering to the subject a pharmaceutical composition comprising a therapeutic effective amount of an anti-inflammatory peptide derived from protease activated receptor-3 (PAR3), wherein the PAR3 derived anti-inflammatory peptide comprises (1) an N-terminal fragment of Met¹-Arg⁴¹ deleted human PAR3 extracellular domain (SEQ ID NO:3) or conservatively modified variant thereof, wherein the fragment consists of at least the first 4 N-terminal residues of SEQ ID NO:3; or (2) at least 4 contiguous amino acid residues of human PAR3 derived peptide P3R (SEQ ID NO:4) or conservatively modified variant thereof, wherein the at least 4 contiguous amino acid residues encompass Phe¹⁰-Phe¹² of SEQ ID NO:4.
**2.** The method of clause 1, wherein the PAR3 derived anti-inflammatory peptide possesses APC-like cytoprotective activities.
**3.** The method of clause 1, wherein the PAR3 derived peptide comprises at least the first 6, 7, 8, 9, 10, 11, 12, 13 or more N-terminal residues of SEQ ID NO:3.
**4.** The method of clause 1, wherein the PAR3 derived peptide comprises GAPPNSFEEFPFS (SEQ ID NO:8) or GAPPNSFEEFPFSALEGWTGATIT (SEQ ID NO:4).
**5.** The method of clause 1, wherein the PAR3 derived peptide comprises at least 6 contiguous amino acid residues of SEQ ID NO:4 encompassing Phe¹⁰-Phe¹².
**6.** The method of clause 5, wherein the PAR3 derived peptide comprises FPFSALEGW (SEQ ID NO:10) or FPFSALEGWT GATIT (SEQ ID NO:9).
**7.** The method of clause 1, wherein the PAR3 derived peptide is conjugated to a carrier moiety.
**8.** The method of clause 7, wherein the carrier moiety is a carrier protein, an immunoglobulin, a Fc domain, or a PEG molecule.
**9.** The method of clause 1, further comprising administering to the subject a therapeutic effective amount of an anti-inflammatory peptide derived from protease activated receptor-1 (PAR1).
**10.** The method of clause 9, wherein the PAR1 derived anti-inflammatory peptide comprises (1) at least the first 4 N-terminal residues of Met¹-Arg⁴¹ deleted human PAR1 extracellular domain (SEQ ID NO:6) or a conservatively modified variant thereof, or (2) a variant of human PAR1 derived peptide TR47 (SEQ ID NO:7) with a deletion or substitution of at least one residue at its N-terminus.
**11.** The method of clause 9, wherein the PAR1 derived peptide comprises the first 4, 5, 6, 7, 8, 9, 10 or more N-terminal residues of human PAR1 derived peptide TR47 (SEQ ID NO:7).
**12.** The method of clause 9, wherein the PAR1 derived peptide comprises a TR47 variant containing a substituted N-terminal residue.
**13.** The method of clause 12, wherein the TR47 variant comprises SEQ ID NO:49 (TR47ΔQ) or SEQ ID NO:50 (TR47ΔA).
**14.** The method of clause 9, wherein the PAR1 derived peptide comprises a TR47 variant containing a deletion of one or more N-terminal residues.
**15.** The method of clause 14, wherein the TR47 variant comprises SEQ ID NO:47 (TR47(N-1)) or SEQ ID NO:48 (TR47(N-5)).
**16.** The method of clause 9, wherein the PAR3 derived peptide and the PAR1 derived peptide are administered to the subject simultaneously.
**17.** The method of clause 16, wherein the administered pharmaceutical composition comprises both the PAR3 derived peptide and the PAR1 derived peptide.
**18.** The method of clause 16, wherein the PAR3 derived peptide is conjugated to the PAR1 derived peptide.
**19.** The method of clause 9, wherein the PAR3 derived peptide and/or the PAR1 derived peptide are conjugated to a carrier moiety.
**20.** The method of clause 19, wherein the ratio of the PAR3-derived peptide and the PAR1-derived peptide is at least about 2:1, 4:1, 6:1, 8:1 or 10:1.
**21.** The method of clause 9, wherein each of the PAR3 derived peptide and the PAR1 derived peptide is administered to the subject at a daily amount of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of the subject's body weight.
**22.** The method of clause 9, wherein the subject is administered with one peptide at a daily amount of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of the subject's body weight, and the other peptide at a daily amount of at least about 0.01 mg/kg, 0.025 mg/kg, 0.05 mg/kg, 0.1 mg/kg, 0.25 mg/ kg. 0.5 mg/kg, 1 mg/kg, 2.5 mg/kg, 5 mg/kg, 10 mg/kg, 25 mg/kg or higher, of the subject's body weight.
**23.** The method of clause 1, wherein the subject is afflicted with or suspected to have an inflammatory disorder selected from the group consisting of asthma, autoimmune diseases, chronic inflammation, chronic prostatitis, gomerulonephritis, hypersensitivities, inflammatory bowel diseases, pelvic inflammatory diseases, reperfusion injury, rheumatoid arthritis, sterile inflammation, transplant rejection, virus-related inflammation, and vasculitis.
**24.** The method of clause 1, wherein the subject is afflicted with or suspected to have a condition associated with undesired immune activation or undesired immune response.
**25.** The method of clause 24, wherein the condition associated with undesired immune activation or undesired immune response is a neuropathology, a viral infection, or malaria.
**26.** The method of clause 1, wherein the subject is afflicted with or suspected to have acute neuroinflammation, chronic neuroinflammation or malarial inflammation.
**27.** A composition comprising a PAR3-derived anti-inflammatory peptide and a PAR1-derived anti-inflammatory peptide.
**28.** The composition of clause 27, wherein (a) the PAR3 derived anti-inflammatory peptide comprises (1) an N-terminal fragment of Met¹-Arg⁴¹ deleted human PAR3 extracellular domain (SEQ ID NO:3) or conservatively modified variant thereof, wherein the fragment consists of at least the first 4 N-terminal residues of SEQ ID NO:3, or (2) at least 4 contiguous amino acid residues of PAR3 derived peptide P3R (SEQ ID NO:4) or conservatively modified variant thereof, wherein the at least 4 contiguous amino acid residues comprises Phe¹⁰-Phe¹² of SEQ ID NO:4; and (b) the PAR1 derived anti-inflammatory peptide comprises (1) an N-terminal fragment of Met¹-Arg⁴⁶ deleted human PAR1 extracellular domain (SEQ ID NO:6) or conservatively modified variant thereof, wherein the fragment consists of at least the first 4 N-terminal residues of SEQ ID NO:6, or (2) a variant of human PAR1 derived peptide TR47 (SEQ ID NO:7) with a deletion or substitution of at least one residue at its N-terminus.
**29.** The composition of clause 27, wherein the PAR3 derived peptide comprises at least the first 13 N-terminal residues of SEQ ID NO:3 or conservatively modified variant thereof, and the PAR1 derived peptide comprises at least the first 8 N-terminal residues of SEQ ID NO:6 or conservatively modified variant thereof.
**30.** The composition of clause 27, wherein the PAR3 derived peptide comprises SEQ ID NO:4, SEQ ID NO:8, SEQ ID NO:9, SEQ ID NO:10, or a conservatively modified variant thereof, and the PAR1 derived peptide comprises SEQ ID NO:7, SEQ ID NO:35, SEQ ID NO:36, SEQ ID NO:43, SEQ ID NO:47, SEQ ID NO:49, SEQ ID NO:50 or a conservatively modified variant thereof.
**31.** The composition of clause 27, wherein the amount of the PAR3-derived peptide and the amount of the PAR1-derived peptide are at a ratio of at least about 2:1, 4:1, 6:1, 8:1 or 10:1.
**32.** The composition of clause 27, wherein the PAR3-derived peptide is conjugated to the PAR1-derived peptide.
**33.** The composition of clause 32, wherein the PAR3-derived peptide is conjugated to the PAR1-derived peptide via a linker moiety or a carrier moiety.
**34.** The composition of clause 27, comprising (a) PAR1-derived peptide comprising the sequence shown in SEQ ID NO:36 or a conservatively modified variant thereof, and (b) PAR3-derived peptide comprising the sequence shown in SEQ ID NO:9 or a conservatively modified variant thereof, wherein (a) and (b) are linked via a peptide moiety.
**35.** The composition of clause 34, comprising the sequence shown in SEQ ID NO:61 or a conservatively modified variant thereof.
**36.** The composition of clause 27, which is formulated for administration to the subject orally, intravenously, subcutaneously, intramuscularly, intranasally, intraocular, topical, or intraperitoneally.
**37.** The composition of clause 27, comprising a daily dosage of each of the two peptides of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of average body weight of the subject group that the composition is intended for.
**38.** The composition of clause 27, comprising (1) a daily dosage of one of the two peptides of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of average body weight of the subject group that the composition is intended for, and (2) a daily dosage of the other peptide of at least about 0.01 mg/kg, 0.025 mg/kg, 0.05 mg/kg, 0.1 mg/kg, 0.25 mg/ kg. 0.5 mg/kg, 1 mg/kg, 2.5 mg/kg, 5 mg/kg, 10 mg/kg, 25 mg/kg or higher, of average body weight of the subject group that the composition is intended for.
**39.** A method for suppressing inflammation and treating inflammatory condition in a subject, comprising administering to the subject a pharmaceutical composition comprising a therapeutic effective amount of an anti-inflammatory peptide derived from protease activated receptor-1 (PAR1), wherein the PAR1 derived anti-inflammatory peptide comprises (1) at least the first 4 N-terminal residues of Met¹-Arg⁴¹ deleted human PAR1 extracellular domain (SEQ ID NO:6) or a conservatively modified variant thereof, or (2) a variant of human PAR1 derived peptide TR47 (SEQ ID NO:7) with a deletion or substitution of at least one residue at its N-terminus.
**40.** The method of clause 39, wherein the PAR1 derived peptide comprises the first 4, 5, 6, 7, 8, 9, 10 or more N-terminal residues of human PAR1 derived peptide TR47 (SEQ ID NO:7).
**41.** The method of clause 39, wherein the PAR1 derived peptide comprises a TR47 variant containing a substituted N-terminal residue.
**42.** The method of clause 41, wherein the TR47 variant comprises SEQ ID NO:49 (TR47ΔQ) or SEQ ID NO:50 (TR47ΔA).
**43.** The method of clause 39, wherein the PAR1 derived peptide comprises a TR47 variant containing a deletion of one or more N-terminal residues.
**44.** The method of clause 43, wherein the TR47 variant comprises SEQ ID NO:47 (TR47(N-1)) or SEQ ID NO:48 (TR47(N-5)).
**45.** A method for identifying an anti-inflammatory agent, comprising (1) culturing a group of human THP-1 cells, (2) contacting the cultured cells with an inflammation inducing agent, (3) contacting the cultured cells respectively with a candidate agent and a PAR3- or PAR1-derived anti-inflammatory peptide, (4) respectively measuring an inflammation related activity in cells that have been contacted with the candidate agent and cells that have been contacted with the PAR3- or PAR1-derived anti-inflammatory peptide; wherein a value of the inflammation activity measured in cells contacted with the candidate agent that is the same or less than a value of the inflammation activity measured in cells contacted with the PAR3- or PAR1-derived anti-inflammatory peptide identifies the candidate agent as an anti-inflammatory agent.
**46.** The method of clause 45, wherein the PAR3- or PAR1-derived anti-inflammatory peptide is P3R (SEQ ID NO:4) or TR47 (SEQ ID NO:7), or a conservatively modified variant thereof.
**47.** The method of clause 45, wherein the candidate agent is a peptide or polypeptide, variant, derivative or mimetic compound of a peptides or polypeptide that mimics the N-terminal sequence of Met¹-Arg⁴¹ deleted extracellular domain of human PAR3 (SEQ ID NO:3) or Met¹-Arg⁴⁶ deleted extracellular domain of human PAR1 (SEQ ID NO:6).
**48.** The method of clause 45, wherein the inflammation related activity is caspase 1 enzymatic activity.
**49.** The method of clause 45, wherein the inflammation related activity is IL-1β release.
**50.** The method of clause 45, wherein the inflammation inducing agent is lipopolysaccharide (LPS).

## Claims

1. A composition comprising a PAR3-derived anti-inflammatory peptide and a PAR1-derived anti-inflammatory peptide.

2. The composition of claim 1, wherein (a) the PAR3 derived anti-inflammatory peptide comprises (1) an N-terminal fragment of Met¹-Arg⁴¹ deleted human PAR3 extracellular domain (SEQ ID NO:3) or conservatively modified variant thereof, wherein the fragment consists of at least the first 4 N-terminal residues of SEQ ID NO:3, or (2) at least 4 contiguous amino acid residues of PAR3 derived peptide P3R (SEQ ID NO:4) or conservatively modified variant thereof, wherein the at least 4 contiguous amino acid residues comprises Phe¹⁰-Phe¹² of SEQ ID NO:4; and (b) the PAR1 derived anti-inflammatory peptide comprises (1) an N-terminal fragment of Met¹-Arg⁴⁶ deleted human PAR1 extracellular domain (SEQ ID NO:6) or conservatively modified variant thereof, wherein the fragment consists of at least the first 4 N-terminal residues of SEQ ID NO:6, or (2) a variant of human PAR1 derived peptide TR47 (SEQ ID NO:7) with a deletion or substitution of at least one residue at its N-terminus.

3. The composition of claim 1, wherein the PAR3 derived peptide comprises at least the first 13 N-terminal residues of SEQ ID NO:3 or conservatively modified variant thereof, and the PAR1 derived peptide comprises at least the first 8 N-terminal residues of SEQ ID NO:6 or conservatively modified variant thereof.

4. The composition of claim 1, wherein the PAR3 derived peptide comprises SEQ ID NO:4, SEQ ID NO:8, SEQ ID NO:9, SEQ ID NO: 10, or a conservatively modified variant thereof, and the PAR1 derived peptide comprises SEQ ID NO:7, SEQ ID NO:35, SEQ ID NO:36, SEQ ID NO:43, SEQ ID NO:47, SEQ ID NO:49, SEQ ID NO:50 or a conservatively modified variant thereof.

5. The composition of claim 1, wherein the amount of the PAR3-derived peptide and the amount of the PAR1-derived peptide are at a ratio of at least about 2:1, 4:1, 6:1, 8:1 or 10:1.

6. The composition of claim 1, wherein the PAR3-derived peptide is conjugated to the PAR1-derived peptide.

7. The composition of claim 6, wherein the PAR3-derived peptide is conjugated to the PAR1-derived peptide via a linker moiety or a carrier moiety.

8. The composition of claim 1, comprising (a) PAR1-derived peptide comprising the sequence shown in SEQ ID NO:36 or a conservatively modified variant thereof, and (b) PAR3-derived peptide comprising the sequence shown in SEQ ID NO:9 or a conservatively modified variant thereof, wherein (a) and (b) are linked via a peptide moiety.

9. The composition of claim 8, comprising the sequence shown in SEQ ID NO:61 or a conservatively modified variant thereof.

10. The composition of claim 1, which is formulated for administration to the subject orally, intravenously, subcutaneously, intramuscularly, intranasally, intraocular, topical, or intraperitoneally.

11. The composition of claim 1, comprising a daily dosage of each of the two peptides of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of average body weight of the subject group that the composition is intended for.

12. The composition of claim 1, comprising (1) a daily dosage of one of the two peptides of at most about 0.25 mg/kg, 0.1 mg/kg, 0.05 mg/kg, 0.025 mg/kg, 0.01 mg/kg, 0.005 mg/kg, 0.0025 mg/kg, 0.001 mg/kg, 0.0005 mg/kg, 0.00025 mg/kg or lower, of average body weight of the subject group that the composition is intended for, and (2) a daily dosage of the other peptide of at least about 0.01 mg/kg, 0.025 mg/kg, 0.05 mg/kg, 0.1 mg/kg, 0.25 mg/ kg. 0.5 mg/kg, 1 mg/kg, 2.5 mg/kg, 5 mg/kg, 10 mg/kg, 25 mg/kg or higher, of average body weight of the subject group that the composition is intended for.

13. A pharmaceutical composition comprising a therapeutic effective amount of an anti-inflammatory peptide derived from protease activated receptor-1 (PAR1), for use in suppressing inflammation and treating inflammatory condition in a subject, wherein the PAR1 derived anti-inflammatory peptide comprises (1) at least the first 4 N-terminal residues of Met¹-Arg⁴⁶ deleted human PAR1 extracellular domain (SEQ ID NO:6) or a conservatively modified variant thereof, or (2) a variant of human PAR1 derived peptide TR47 (SEQ ID NO:7) with a deletion or substitution of at least one residue at its N-terminus.

14. The composition for use according to claim 13, wherein the PAR1 derived peptide comprises the first 4, 5, 6, 7, 8, 9, 10 or more N-terminal residues of human PAR1 derived peptide TR47 (SEQ ID NO:7).

15. The composition for use according to claim 13, wherein the PAR1 derived peptide comprises a TR47 variant containing a substituted N-terminal residue.
